# EUROPEAN PATENT SPECIFICATION

(11) **EP 3 054 227 B1**
(45) Date of publication and mention of the grant of the patent: **04.09.2019**
(21) Application number: 14849034.5
(22) Date of filing: 25.09.2014
(51) Int. Cl.: F24F 7/00, A61L 9/00, F24F 6/06, F24F 13/24, F24F 3/16, F24F 13/28

(54) **AIR PURIFIER**
LUFTREINIGER
PURIFICATEUR D'AIR

(30) Priority: 30.09.2013 JP 2013205507; 04.07.2014 JP 2014139205
(43) Date of publication of application: 10.08.2016
(73) Proprietor: Daikin Industries, Ltd., Osaka-shi, Osaka 530-8323 (JP)
(72) Inventor: SAKASHITA, Akihiko, Osaka-shi Osaka 530-8323 (JP); NAGAO, Mitsuhisa, Osaka-shi Osaka 530-8323 (JP); ODA, Yasuhiro, Osaka-shi Osaka 530-8323 (JP); HANAOKA, Sanae, Osaka-shi Osaka 530-8323 (JP); SUOU, Kiyoyuki, Osaka-shi Osaka 530-8323 (JP)
(74) Representative: Goddar, Heinz J.
(86) International application number: PCT/JP2014/075508
(87) International publication number: WO 2015/046368

(56) References cited:
- JP-A- S62 742
- JP-A- S62 742
- JP-A- H06 288 567
- JP-A- H06 288 567
- JP-A- 2003 028 486
- JP-A- 2003 083 574
- JP-A- 2004 085 080
- JP-A- 2009 041 835
- JP-A- 2010 151 365
- JP-U- S5 682 430
- US-A1- 2012 234 166

## Description

### TECHNICAL FIELD

The present invention relates to an air purifier, particularly to an air purifier with a humidification function.

### BACKGROUND ART

In recent years, air purifiers with a humidification function as described in PTL 1 (Japan Laid-open Patent Application Publication No. 2010-17685) have been widely prevailed. In this type of air purifiers, an air purification filter, a humidification element and a fan are disposed in this order from the upstream side in the depth direction of air purifier body.

JPH06288567 discloses an air purifier according to the preamble of claim 1.

### SUMMARY OF THE INVENTION

### <Technical Problem>

Generally in the type of air purifiers as described in PTL 1 (Japan Laid-open Patent Application Publication No. 2010-17685), a tendency is observed that in a high power operation, the sound of the fan is likely to leak out through a blow-out port along the flow of air.

In view of this, it is an object of the present invention to provide an air purifier in which fan-attributed noises are inhibited.

### <Solution to Problem>

An air purifier according to a first aspect of the present invention is an air purifier having a humidity regulation function, and includes an air purification filter, a humidification element and a fan. The air purification filter is configured to remove dust and dirt contained in air. The humidification element is configured to humidify the air by vaporizing water to be supplied thereto. The fan is configured to blow the air to the air purification filter and the humidification element. Additionally, the fan, the air purification filter and the humidification element are sequentially disposed from below in an order of the fan, the air purification filter and the humidification element.

In the present air purifier, the air sucked from below by the fan passes through the air purification filter located in a high position, then passes through the humidification element located in a higher position, and is blown out from the air purifier. The fan as a sound source is located in the lowermost position. Hence, similarly to the flow of air, the sound of the fan tends to propagate from a lower side to a higher side. Therefore, when the power of the fan is high, the sound produced from the fan is attenuated by the air purification filter and the humidification filter. In other words, noises are inhibited. Additionally, a body casing has a vertically elongated shape, and is therefore installed in a small area. Moreover, the humidification element, requiring a maintenance work, is located in a higher position. Hence, the maintenance work is easily performed.

An air purifier according to a second aspect of the present invention relates to the air purifier according to the first aspect, and further includes a body casing. The body casing accommodates the air purification filter, the humidification element and the fan. Additionally, the body casing includes a blowing compartment, an air purification compartment, a humidification compartment, a first opening and a second opening. The fan is disposed in the blowing compartment. The air purification filter is disposed in the air purification compartment. The humidification element is disposed in the humidification compartment. The first opening is an opening through which the air flows from the blowing compartment to the air purification compartment. The second opening is an opening through which the air flows from the air purification compartment to the humidification compartment. Additionally, the first opening and the second opening are displaced from each other in a top view.

In general, static pressure acts on the entire surface of the air purification filter by the air that has entered the air purification compartment, and the air passes through the air purification filter. At this time, the speed of air becomes high in a part opposed to the first opening and a part opposed to the second opening in the air purification filter. When it is assumed that the first opening and the second opening widely overlap with each other in a top view, air flows at a high speed in passing through the air purification filter at a part corresponding to the overlapped region. Thus, the speed distribution of air exhibits a tendency that the speed of air is remarkably high in the overlapped region whereas air does not pass through or the speed of air is extremely low in positions far from the overlapped region. However, in the present air purifier, the first opening and the second opening are displaced from each other in the top view, and thereby, the overlapped region is reduced or eliminated. Hence, the speed of air exhibits a widely ranged speed distribution when air passes through the air purification filter. In other words, a part, through which air does not pass or in which the speed of air is extremely low, is reduced in the air purification filter.

An air purifier according to a third aspect of the present invention relates to the air purifier according to the second aspect, and wherein the first opening is displaced to one side with respect to a center of the air purification filter, and the second opening is displaced oppositely to the one side with respect to the center of the air purification filter.

An air purifier according to a fourth aspect of the present invention relates to the air purifier according to the second aspect, and further includes a fan motor, which is disposed in the blowing compartment and is configured to drive the fan, and a water storage tank, which is disposed in the humidification compartment and is configured to supply the water to the humidification element The fan motor is displaced to one side with respect to a center of the air purification filter, and the water storage tank is displaced oppositely to the one side with respect to the center of the air purification filter.

In the present air purifier, the body thereof has better upright stability when the heavy fan motor and the heavy tank are separated away from each other in opposite (right-and-left or back-and-forth) directions rather than when they are disposed only on the same side in a top view.

An air purifier according to a fifth aspect of the present invention relates to the air purifier according to the second aspect, and wherein the air purification filter has a horizontal projected area of greater than or equal to 80% of a horizontal projected area of the air purification compartment. The first opening has an area set to fall in a range of 20% to 30% of the horizontal projected area of the air purification compartment. The second opening has an area set to fall in a range of 40% to 50% of the horizontal projected area of the air purification compartment

The air blown out from the fan passes through the first opening. Hence, in general, air is likely to flow at a high speed in passing through the air purification filter at a part opposed to the first opening. Therefore, the speed of air tends to exhibit a speed distribution that the speed is extremely high in part of the entire range. However, in the present air purifier, the overlapped region between the first opening and the second opening is eliminated or reduced by setting the second opening to be larger than the first opening and simultaneously by displacing the first opening and the second opening in a top view. Hence, the speed of air exhibits a widely ranged speed distribution when air passes through the air purification filter, and a part, through which air does not pass or in which the speed of air is extremely low, is reduced in the air purification filter.

An air purifier according to a sixth aspect of the present invention relates to the air purifier according to the first aspect, and wherein the fan is a sirocco fan. Therefore, a stable airflow rate is achieved.

An air purifier according to a seventh aspect of the present invention relates to the air purifier according to any of the first to sixth aspects, and further includes an airflow guide disposed above the air purification filter. The airflow guide includes a first guide surface with a curved shape configured to direct the air having passed through the air purification filter to the humidification element

In the present air purifier, with the airflow guide herein provided, the air having passed through the air purification filter can be easily and smoothly directed to the humidification element, and also, the air can be directed to the humidification element while inhibiting pressure loss.

An air purifier according to an eighth aspect of the present invention relates to the air purifier according to the seventh aspect, and wherein the airflow guide is integrated with a filter case accommodating at least part of the air purification filter.

In the present air purifier, the filter case accommodating the air purification filter and the airflow guide are integrated. Hence, the air having passed through the air purification filter can be easily directed to the humidification element without being allowed to flow through another path.

An air purifier according to a ninth aspect relates to the air purifier according to any of the first to eighth aspects, and further includes a water retention tray which is disposed below the humidification element and is configured to retain the water to be supplied to the humidification element The water retention tray includes a second guide surface with a curved shape configured to direct the air having passed through the air purification filter to the humidification element, on a lower part thereof.

In the present air purifier, with the second guide surface herein provided on the lower part of the water retention tray, the air having passed through the air purification filter can be easily and smoothly directed to the humidification element, and also, the air can be directed to the humidification element while inhibiting pressure loss.

An air purifier according to a tenth aspect of the present invention relates to the air purifier according to the seventh or eighth aspect, and further includes a water retention tray which is disposed below the humidification element and is configured to retain the water to be supplied to the humidification element. The water retention tray includes a second guide surface with a curved shape configured to direct the air having passed through the air purification filter to the humidification element, on a lower part thereof. The first guide surface and the second guide surface form a continuous curve through a gap produced therebetween.

In the present air purifier, with a continuous curve formed by the first and second guide surfaces, the air having passed through the air purification filter can be easily and smoothly directed to the humidification element, and also, the air can be directed to the humidification element while inhibiting pressure loss.

An air purifier according to an eleventh aspect of the present invention relates to the air purifier according to the seventh or eighth aspect, and further includes a water retention tray which is disposed below the humidification element and is configured to retain the water to be supplied to the humidification element The airflow guide includes an extended part shaped for covering a second opening-side lower part of the water retention tray. The extended part includes an extended guide surface with a curved shape configured to direct the air having passed through the air purification filter to the humidification element. The extended guide surface forms a continuous curve together with the first guide surface.

In the present air purifier, the second opening-side lower part of the water retention tray is covered with the airflow guide, and the airflow guide includes the first guide surface and the extended guide surface that form a continuous curve. Therefore, the air having passed through the air purification filter can be directed to the humidification element while inhibiting pressure loss, without being allowed to flow through the gap produced between the airflow guide and the water retention tray.

An air purifier according to a twelfth aspect of the present invention relates to the air purifier according to the second aspect, and wherein the body casing includes a bypass opening bored above the second opening so as to direct the air to outside of the body casing from the humidification compartment without passing the air through the humidification element.

In the present air purifier, the bypass opening is bored above the second opening, and hence, part of air can be directed outside the body casing through the bypass opening, while pressure loss is hardly caused. Therefore, it is easy to make air flow through the air purification filter at a large airflow rate. Moreover, the first opening and the second opening are herein displaced from each other in a top view. Hence, it is possible to make air evenly flow through the entire surface of the air purification filter at a large airflow rate, and thus, it is possible to efficiently remove dust and dirt from air.

### <Advantageous Effects of Invention>

In the air purifier according to the first aspect of the present invention, the air sucked from below by the fan passes through the air purification filter located in a high position, then passes through the humidification element located in a higher position, and is blown out from the air purifier. The fan as a sound source is located in the lowermost position. Hence, similarly to the flow of air, the sound of the fan tends to propagate from a lower side to a higher side. Therefore, when the power of the fan is high, the sound produced from the fan is attenuated by the air purification filter and the humidification filter. In other words, noises are inhibited. Additionally, the body casing has a vertically elongated shape, and is therefore installed in a small area. Moreover, the humidification element, requiring a maintenance work, is located in a higher position. Hence, the maintenance work is easily performed.

In the air purifier according to the second or third aspect of the present invention, the first opening and the second opening are displaced from each other in a top view, and thereby, the overlapped region between the first opening and the second opening is reduced or eliminated. Hence, the speed of air exhibits a widely ranged speed distribution when air passes through the air purification filter. In other words, a part, through which air does not pass or in which the speed of air is extremely low, is reduced in the air purification filter.

In the air purifier according to the fourth aspect of the present invention, the body thereof has better upright stability when the heavy fan motor and the heavy tank are separated away from each other in opposite (right-and-left or back-and-forth) directions rather than when they are disposed only on the same side in a top view.

In the air purifier according to the fifth aspect of the present invention, the overlapped region between the first opening and the second opening is eliminated or reduced by setting the second opening to be larger than the first opening and simultaneously by displacing the first opening and the second opening in a top view. Hence, the speed of air exhibits a widely ranged speed distribution when air passes through the air purification filter, and a part, through which air does not pass or in which the speed of air is extremely low, is reduced in the air purification filter.

In the air purifier according to the sixth aspect of the present invention, the fan is a sirocco fan. Therefore, a stable airflow rate is achieved.

In the air purifier according to the seventh aspect of the present invention, the air having passed through the air purification filter can be easily and smoothly directed to the humidification element, and also, the air can be directed to the humidification element while inhibiting pressure loss.

In the air purifier according to the eighth aspect of the present invention, the filter case and the airflow guide are integrated. Hence, the air having passed through the air purification filter can be easily directed to the humidification element without being allowed to flow through another path.

In the air purifier according to the ninth or tenth aspect of the present invention, the air having passed through the air purification filter can be easily and smoothly directed to the humidification element, and also, the air can be directed to the humidification element while inhibiting pressure loss.

In the air purifier according to the eleventh aspect of the present invention, the air having passed through the air purification filter can be directed to the humidification element while inhibiting pressure loss, without being allowed to flow through the gap produced between the airflow guide and the water retention tray.

In the air purifier according to the twelfth aspect of the present invention, it is possible to make air evenly flow through the entire surface of the air purification filter at a large airflow rate, and thus, it is possible to efficiently remove dust and dirt from the air.

### BRIEF DESCRIPTION OF THE DRAWINGS

FIG. 1 is a perspective view of an air purifier according to a first embodiment of the present invention seen from an obliquely front side.
FIG. 2 is a cross-sectional view of the air purifier.
FIG. 3 is a horizontal projection of a first opening and a second opening in the air purifier.
FIG. 4 is a horizontal projection of a first opening and a second opening in an air purifier constructed more compactly than the air purifier of FIG 3.
FIG. 5A is a distribution chart of the speed of air that has passed through an air purification filter in a configuration that the first opening and the second opening are displaced in a top view as shown in FIG 3.
FIG. 5B is a distribution chart of the speed of air that has passed through an air purification filter in a configuration that the second opening completely overlaps with the first opening in a top view.
FIG. 6 is a perspective view of an air purifier according to a second embodiment of the present invention.
FIG. 7 is a cross-sectional view of the air purifier taken along arrow VII-VII in FIG 6, and is also a view of the interior of the air purifier seen from the right side.
FIG. 8 is a cross-sectional view of the air purifier taken along arrow VIII-VIII in FIG. 6, and is also a view of the interior of the air purifier seen from the front side.
FIG. 9 is a top view of a humidification element and its surroundings contained in a casing of the air purifier shown in FIG. 6.
FIG 10 is a drawing for explaining a structure of a fan and its surroundings in the air purifier shown in FIG 6 and flow directions of air to be blown out from the fan.
FIG. 11 is a plan view of a dust collecting filter to be used for the air purifier shown in FIG. 6.

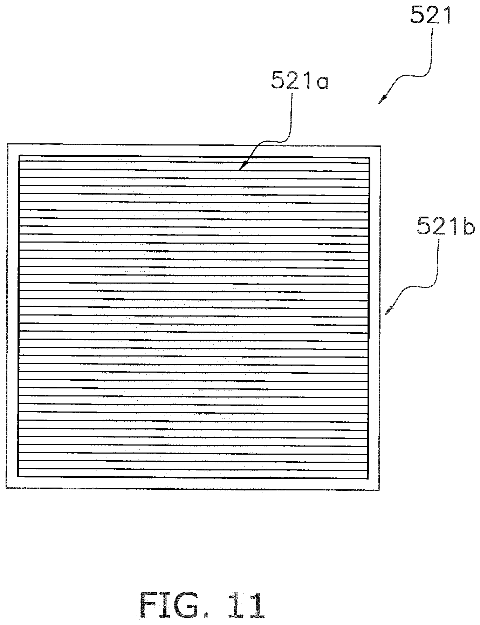

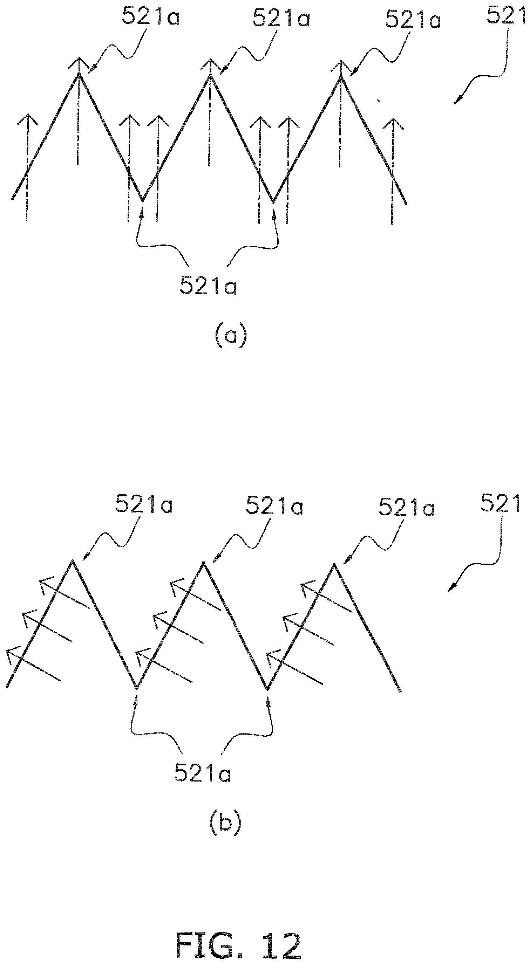

FIG 12 is a drawing for schematically showing directions of air passing through the dust collecting filter shown in FIG 11, and includes: diagram (a) schematically showing the directions of air passing through the dust collecting filter in a configuration that the extending direction of creases of the dust collecting filter and the extending direction of a tongue of a scroll casing (direction of the rotational axis of the fan) are orthogonal to each other; and diagram (b) schematically showing the directions of air passing through the dust collecting filter in a configuration that the extending direction of the creases of the dust collecting filter and the extending direction of the tongue of the scroll casing are parallel to each other.
FIG 13 is a perspective view of a filter case of the air purifier shown in FIG 6 seen from the left front side, and shows a condition that a deodorization filter is accommodated in the filter case.
FIG. 14 is a perspective view of the filter case shown in FIG 13 seen from the right front side.
FIG 15 is a drawing for explaining a deodorization filter detection sensor arranged adjacently to the filter case shown in FIG. 13, and includes: diagram (a) showing a condition of the deodorization filter detection sensor when the deodorization filter is not accommodated in the filter case; and diagram (b) showing a condition of the deodorization filter detection sensor when the deodorization filter is accommodated in the filter case.
FIG. 16 is a drawing for explaining attachment of a humidification tray to the casing in the air purifier shown in FIG. 6 and coupling of a rotor rotating shaft of the humidification element and a drive shaft of a humidification motor.
FIG 17 is a drawing for explaining the flow of air in the air purifier shown in FIG 6, and explains the flow of air with a cross-sectional view of the interior of the casing seen from the front side.
FIG. 18 is a drawing for explaining the flow of air in the air purifier shown in FIG 6, particularly in the surroundings of a humidification unit and bypass openings, and the humidification element is not illustrated herein.

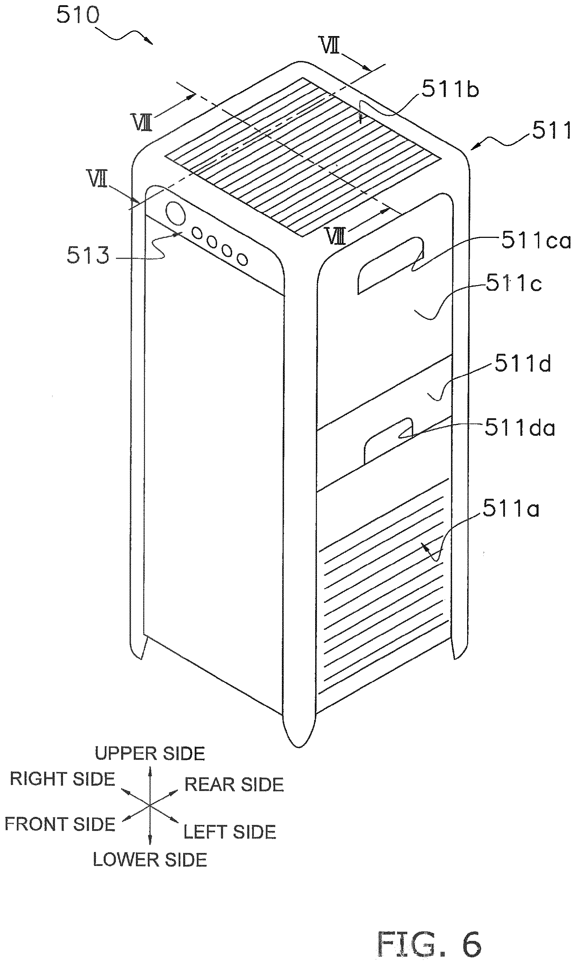

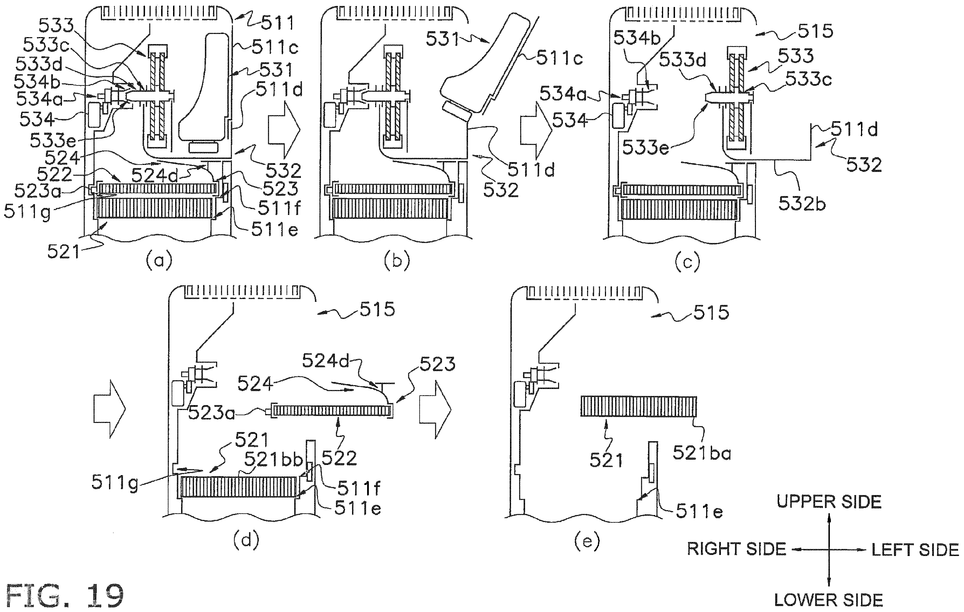

FIG. 19 is a cross-sectional view of the air purifier shown in FIG. 6 seen from the front side illustrating a process of detaching the humidification unit and the air purification filter, and includes: diagram (a) showing a condition before the humidification unit and the air purification filter are detached; diagram (b) showing a condition that a humidification tank of the humidification unit is being detached; diagram (c) showing a condition that the humidification tray of the humidification unit is being detached; diagram (d) showing a condition that the deodorization filter of the air purification filter is being detached; and diagram (e) showing a condition that the dust collecting filter of the air purification filter is being detached.
FIG 20 is a schematic perspective view of the air purifier shown in FIG 6 seen from the left front side illustrating a process of detaching the humidification unit, and includes: diagram (a) showing a condition before the humidification unit is detached; diagram (b) showing a condition that the humidification tank is being detached; diagram (c) showing a condition that the humidification tank has been detached; and diagram (d) showing a condition that the humidification tray is being detached.
FIG. 21 is a drawing schematically depicting a condition of the air purifier shown in FIG 6 that the filter case has been attached thereto without attaching the dust collecting filter thereto.
FIG 22 is a cross-sectional view of the interior of an air purifier according to Modification F seen from the right side, and a configuration of a humidification rotor ladle type is employed for a humidification unit in the air purifier according to Modification F.
FIG 23 is a perspective view of a filter case of an air purifier according to Modification G seen from the left front side in a condition that a deodorization filter is accommodated in the filter case.
FIG. 24 is a cross-sectional view of the interior of the air purifier according to Modification G seen from the front side.

### DESCRIPTION OF EMBODIMENTS

Embodiments of the present invention will be hereinafter explained with reference to drawings. It should be noted that the following embodiments are specific examples of the present invention, and are not intended to limit the technical scope of the present invention.

### <First Embodiment>

### (1) Entire Configuration of Air Purifier 10

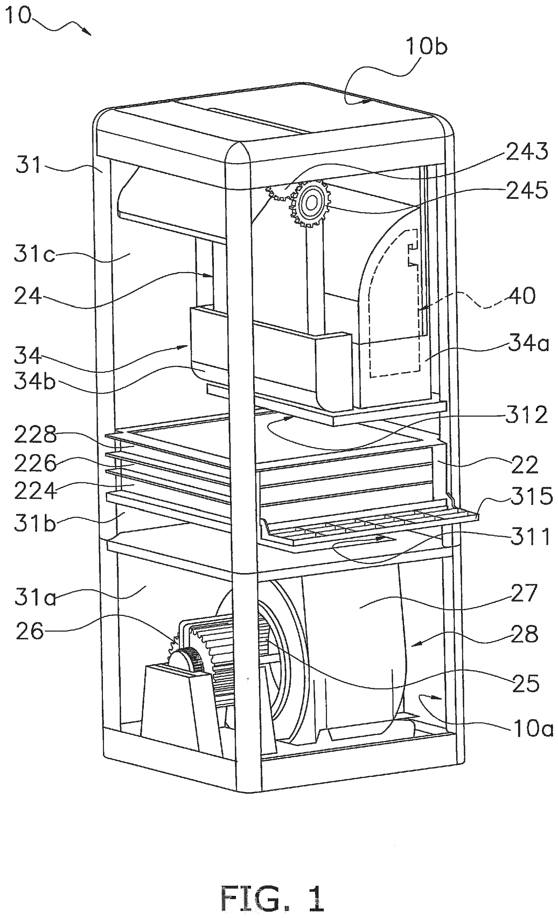

FIG. 1 is a perspective view of an air purifier 10 according to a first embodiment of the present invention seen from an obliquely front side. It should be noted that FIG 1 is illustrated with a condition that four vertical lateral surfaces are removed to make the internal structure visible.

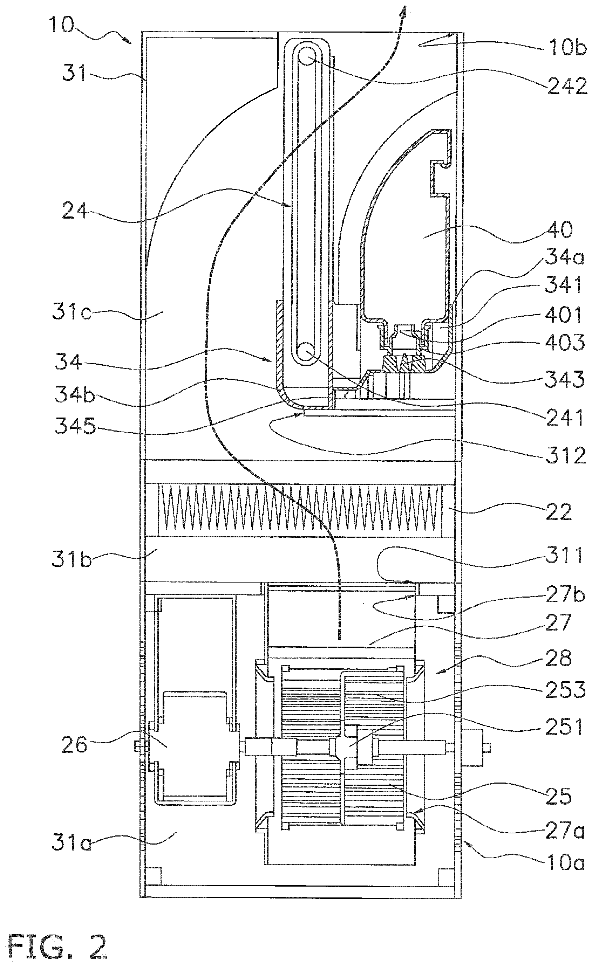

FIG. 2 is a cross-sectional view of the air purifier 10. In FIGS. 1 and 2, the air purifier 10 includes a body casing 31 made in the shape of a rectangular prism. The air purifier 10 has a humidification function and an air purification function. A user can select only the air purification function. However, when the humidification function is selected, the air purification function is selected simultaneously.

### (2) Detailed Configuration

### (2-1) Body Casing 31

The body casing 31 includes a blowing compartment 31a, an air purification compartment 31b and a humidification compartment 31c. These compartments are aligned from bottom to top in the order of the blowing compartment 31a, the air purification compartment 31b and the humidification compartment 31c. In other words, the body casing 31 has a vertically elongated shape, and is therefore installed in a small area.

A blower device 28 is disposed in the blowing compartment 31a. An air purification filter 22 is disposed in the air purification compartment 31b. A humidification element 24, a tray 34 and a tank 40 are disposed in the humidification compartment 31c. In other words, the humidification element 24, requiring a maintenance work, is located in a higher position. Hence, the maintenance work is easily performed. It should be noted that the tank 40 is designed to be installable and removable through a lateral door (not shown in the drawings).

A first opening 311 is provided between the blowing compartment 31a and the air purification compartment 31b. The first opening 311 enables air to flow therethrough from the blowing compartment 31a to the air purification compartment 31b. Moreover, a second opening 312 is provided between the air purification compartment 31b and the humidification compartment 31c. The second opening 312 enables air to flow therethrough from the air purification compartment 31b to the humidification compartment 31c.

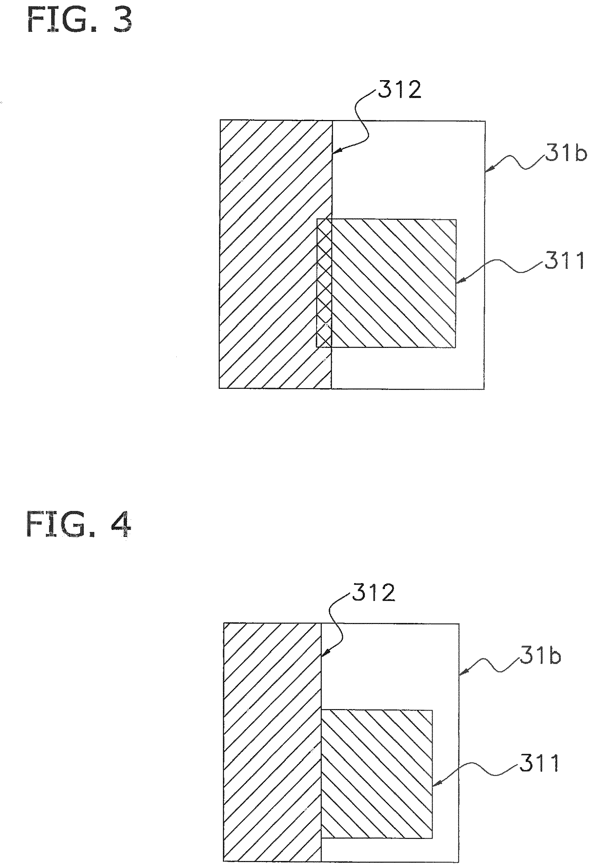

FIG 3 is a horizontal projection of the first opening 311 and the second opening 312 in the air purifier 10. FIG. 4 is a horizontal projection of the first opening 311 and the second opening 312 in an air purifier constructed more compactly than the air purifier 10 of FIG 3.

In each of FIGS. 3 and 4, a square drawn outside indicates the contour of the air purification compartment 31b. A rectangle, located on the left side in a top view within the contour of the air purification compartment 31b, indicates the second opening 312. Moreover, a rectangle, located next to the second opening 312, indicates the first opening 311.

The area of the second opening 312 is set to fall in a range of 40% to 50% of the horizontal projected area of the air purification compartment 31b. The area of the first opening 311 is set to fall in a range of 20% to 30% of the horizontal projected area of the air purification compartment 31b.

In each of FIGS. 3 and 4, the first opening 311 and the second opening 312 are displaced in opposite directions in the top view, and the overlapping area between the first opening 311 and the second opening 312 in the top view is purposefully set to be reduced or eliminated.

### (2-2) Blower Device 28

The blower device 28 includes a fan rotor 25, a fan motor 26 and a scroll member 27. The fan rotor 25 is a sirocco fan in which a stable airflow rate is achieved and, as shown in FIG. 2, includes a hub unit 251 and a plurality of vanes 253 that are cylindrically aligned on the circumferential edge of the hub unit 251. When the hub unit 251 and the vanes 253 are rotated, air is sucked along the direction of the rotational axis of the hub unit 251 and is blown out from the vanes 253 in centrifugal directions.

The scroll member 27 forms an air passage for directing the air blown out from the vanes 253 to a fan blow-out port 27b located above the fan rotor 25. The fan blow-out port 27b is fitted to the first opening 311 being located between the blowing compartment 31a and the air purification compartment 31b. Hence, the air blown out from the fan blow-out port 27b enters the air purification compartment 31b.

### (2-3) Air Purification Filter 22

As shown in FIG 1, the air purification filter 22 is composed of a pre-filter 224, a filter 226 and a deodorization element 228. These constituent elements are held in the air purification compartment 31b, while being aligned sequentially from the upstream side of the flow of air in the order of the pre-filter 224, the filter 226 and the deodorization element 228.

The pre-filter 224 is a thin soft net made of resin, and removes large particle dust and so forth contained in air. The filter 226 removes fine dust and so forth that cannot be removed by the pre-filter 224. The deodorization element 228 absorbs unpleasant odor components contained in air.

It should be noted that in the present embodiment, a hinged open/close door 315 is provided on one of the four lateral surfaces of the air purification compartment 31b in order to make it easy to take out the air purification filter 22 in performing a maintenance work of the air purification filter 22.

### (2-4) Humidification Element 24

The humidification element 24 is disposed above the tray 34. The humidification element 24 is shaped in a loop, and is supported from inside by two rollers vertically disposed away from each other such that a predetermined tension acts thereon.

For convenience of explanation, the lower one of the two rollers is referred to as a first roller 241, whereas the upper one of the two rollers is referred to as a second roller 242. The first roller 241, together with the lower part of the humidification element 24, is immersed into water in the tray 34.

A driven gear 245 is fixed to a position extended from the rotational axis of the second roller 242. The driven gear 245 meshes with a drive gear 243. The drive gear 243 is fixed to a rotational shaft of a drive motor (not shown in the drawings). With the positional relation, the drive gear 243 and the driven gear 245 are configured to be disengaged from each other in taking out the tray 34 from the tank 40 side. Contrarily, the drive gear 243 and the driven gear 245 are configured to be engaged with each other in inserting the tray 34 from the tank 40 side.

When the drive motor is rotated, the torque thereof is sequentially transmitted to the drive gear 243, the driven gear 245 and the second roller 242, and accordingly, the humidification element 24 is circulated.

### (2-5) Tray 34

The tray 34 includes a tank receiver 34a and a water receiver 34b. The tank receiver 34a is provided with a support part 341 and a push pin 343. The support part 341 supports a predetermined corner of the tank 40 mounted thereto with a water feed valve being directed downward. When the tank 40 moves down by its own weight, the push pin 343 makes contact with and opens the water feed valve.

Water is retained in the water receiver 34b in order to immerse the humidification element 24 therein. The water receiver 34b and the tank receiver 34a are divided by a partition 345, but the partition 345 has a cutout. The water, flowing out from the tank 40 to the tank receiver 34a, flows to the water receiver 34b through the cutout. Thus, the water receiver 34b is filled with water to a predetermined level.

### (2-6) Tank 40

A water feed valve 403 is attached to a water filling port 401 of the tank 40. The water feed valve 403 has a general structure that a valve element is pushed onto a valve port by a spring. Therefore, detailed explanation thereof will not be provided herein.

In a normal use condition, the tank 40 is disposed on the tank receiver 34a of the tray 34, and is pushed onto the push pin 343 of the tray 34, with the water feed valve 403 being directed vertically downward. Hence, the push pin 343 opens the water feed valve 403 due to the weight of the tank 40.

### (2-7) Others

In FIG. 2, the fan motor 26, which is a heavy object, is disposed on the left side in a front view of FIG 2 within the blowing compartment 31a. On the other hand, the tank 40, which is also a heavy object, is disposed on the right side of the front view of FIG. 2 within the humidification compartment 31c. In other words, the heavy fan motor 26 and the heavy tank 40 are separated away from each other in opposite (right-and-left or back-and-forth) directions without being disposed only on the same side in a top view. Thus, a good weight balance is achieved.

### (3) Action of Air Purifier 10

### (3-1) Air Purification Action

An action of the air purifier 10 constructed as described above will be hereinafter explained. When the air purifier 10 is powered on, the fan motor 26 of the blower device 28 is configured to rotate the fan rotor 25. The fan rotor 25 in a rotary motion blows out air in the centrifugal directions. Hence, pressure decreases in the surroundings of the rotational axis of the fan rotor 25. Accordingly, air is sucked into a fan suction port 27a located in the center of the fan rotor 25. As a result, the flow of air, directed from a suction port 10a to the fan suction port 27a, is generated.

The air blown out from the fan rotor 25 in the centrifugal directions flows toward the fan blow-out port 27b while being deflected along the scroll member 27. The fan blow-out port 27b is fitted to the first opening 311. Hence, the air blown out from the fan blow-out port 27b enters the air purification compartment 31b.

When air passes through the air purification filter 22, relatively large particle dust and so forth, which are contained in the air, are firstly removed by the pre-filter 224 disposed on the upstream side, and then, small particle dust is removed from the air by the filter 226 disposed on the downstream side. When the air, having passed through the filter 226, passes through the deodorization element 228 disposed on the further downstream side, unpleasant odor components contained in air are absorbed by the deodorization element 228. The air, having passed through the air purification filter 22, passes through the second opening 312 and enters the humidification compartment 31c.

Here, in the air purification compartment 31b, static pressure acts on the entire surface of the air purification filter 22 by the air flowing from the blowing compartment 31a, and the air passes through the air purification filter 22. At this time, air increases in speed in flowing through the air purification filter 22 at a part opposed to the first opening 311.

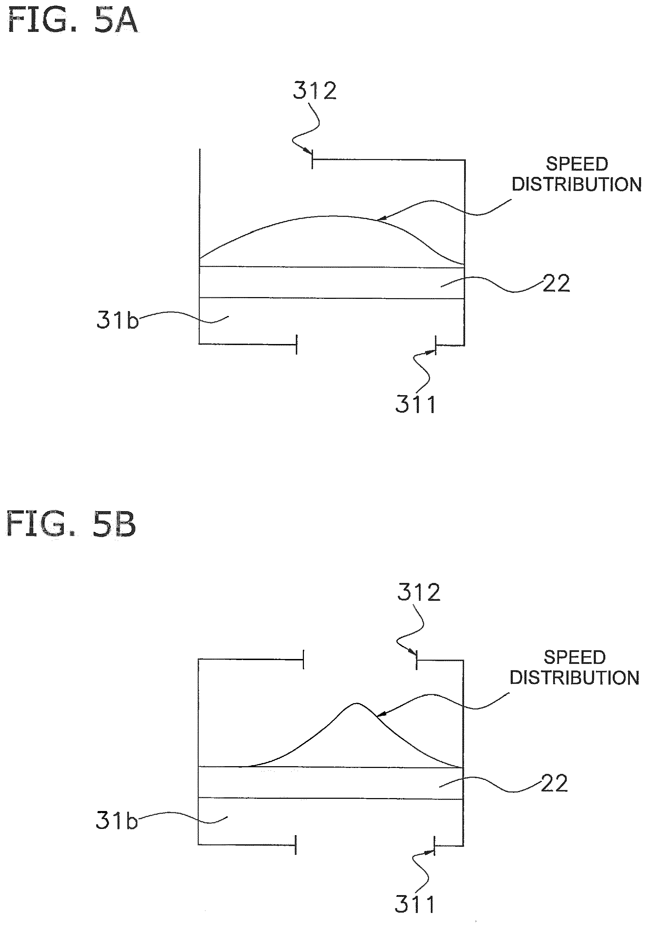

FIG. 5A is a distribution chart of the speed of the air having passed through the air purification filter 22 in a configuration that the first opening 311 and the second opening 312 are displaced in a top view as shown in FIG. 3. On the other hand, FIG. 5B is a distribution chart of the speed of the air having passed through the air purification filter 22 in a configuration that the second opening 312 completely overlaps with the first opening 311 in a top view.

Suppose that the first opening 311 and the second opening 312 completely overlap with each other in a top view, as shown in FIG. 5B, air flows at a high speed in passing through the air purification filter 22 at a part corresponding to the overlapped region. Thus, the speed distribution of air exhibits a tendency that the speed of air is remarkably high in the overlapped region whereas air does not pass through or the speed of air is extremely low in positions far from the overlapped region.

However, in the air purifier 10 as shown in FIGS. 3 and 4, the first opening 311 and the second opening 312 are displaced from each other in the top view, and thereby, the overlapped region is reduced or eliminated. Hence, as shown in FIG 5A, the speed of air exhibits a widely ranged distribution when air passes through the air purification filter 22. In other words, a part, through which air does not pass or in which the speed of air is extremely low, is reduced in the air purification filter 22.

### (3-2) Humidification Action

When the humidification function is turned on in the powered-on state of the air purifier 10, the humidification element 24 is configured to be circulated. The lower part of the humidification element 24 is immersed into the water in the tray 34. Hence, the water in the tray 34 is configured to be absorbed by the circulation of the humidification element 24.

The air, having entered the humidification compartment 31c from the air purification compartment 31b, promotes vaporization of the water absorbed by the humidification element 24, and humidified air is produced. The humidified air is blown out from a blow-out port 10b.

### (4) Features

### (4-1)

In the present air purifier 10, air sucked by the fan rotor 25 from the blowing compartment 31a located in a low position passes through the air purification filter 22 disposed in the air purification compartment 31b located in a high position, then passes through the humidification element 24 disposed in the humidification compartment 31c located in a higher position, and is blown out from the air purifier 10. The blower device 28 as a sound source is located in the lowermost position. Therefore, when the power of the fan motor 26 is high, sounds produced from the fan motor 26 and the fan rotor 25 are attenuated by the air purification filter 22 and the humidification element 24. In other words, noises are inhibited.

### (4-2)

In the present air purifier 10, static pressure acts on the entire surface of the air purification filter 22 by the air that has entered the air purification compartment 31b, and the air passes through the air purification filter 22. The first opening 311 between the blowing compartment 31a and the air purification compartment 31b and the second opening 312 between the air purification compartment 31b and the humidification compartment 31c are displaced from each other in a top view. Hence, in comparison with a configuration that these openings are not displaced from each other, the speed of air exhibits a widely ranged speed distribution when air passes through the air purification filter 22, and a part, through which air does not pass or in which the speed of air is extremely low, is reduced in the air purification filter 22.

### (4-3)

The heavy fan motor 26 and the heavy tank 40 are separated away from each other in opposite (right-and-left or back-and-forth) directions without being disposed only on the same side in a top view. Thus, the body casing 31 has good upright stability.

### (4-4)

The air blown out from the fan blow-out port 27b passes through the first opening 311. Hence, air is likely to flow at a high speed in passing through the air purification filter 22 at a part opposed to the first opening 311. Therefore, the speed of air tends to exhibit a speed distribution that the speed is extremely high in part of the entire range. However, the overlapped region between the first opening 311 and the second opening 312 is eliminated or reduced by setting the second opening 312 to be larger than the first opening 311 and simultaneously by displacing the first opening 311 and the second opening 312 in a top view. Hence, the speed of air exhibits a widely ranged speed distribution when air passes through the air purification filter 22, and a part, through which air does not pass or in which the speed of air is extremely low, is reduced in the air purification filter 22.

### <Second Embodiment>

An air purifier 510 according to a second embodiment of the present invention will be hereinafter explained.

### (1) Entire Configuration of Air Purifier

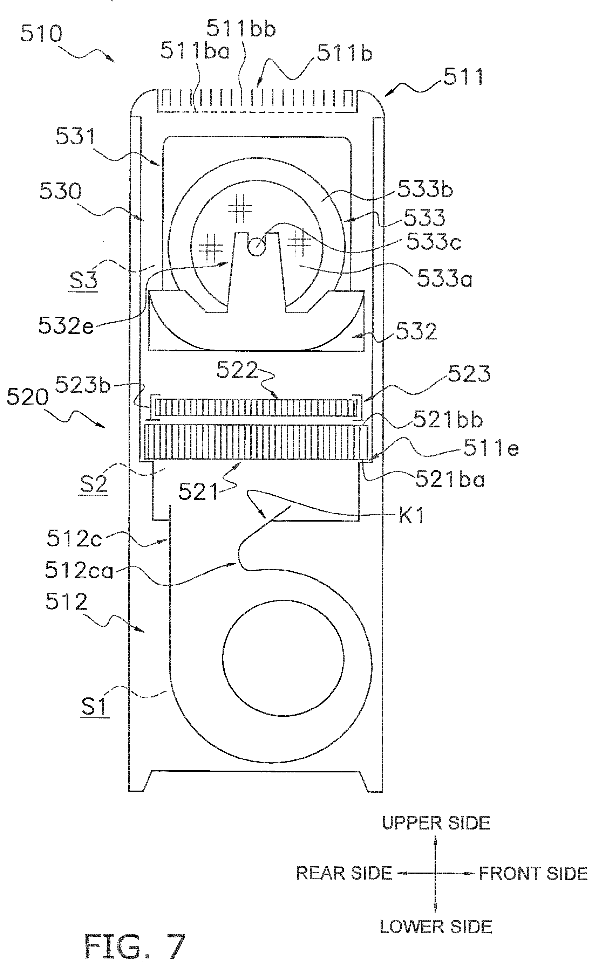

FIG 6 is a perspective view of the appearance of the air purifier 510 according to the second embodiment of the present invention. In the following explanation, expressions such as "front (front side)", "rear (rear side)", "right", "left", "up" and "down" will be used on an as-needed basis for explaining directions, positional arrangements and so forth. Unless otherwise stated, the directions, positional arrangements and so forth are expressed based on arrows depicted in FIG 6. It should be noted that arrows indicating "front", "rear", "right", "left", "up" and "down" are also depicted in FIGS. 7 to 9 and FIGS. 17 to 19. The directions indicated by the arrows in FIGS. 7 to 9 and FIGS. 17 to 19 respectively correspond to those indicated by the arrows in FIG. 6.

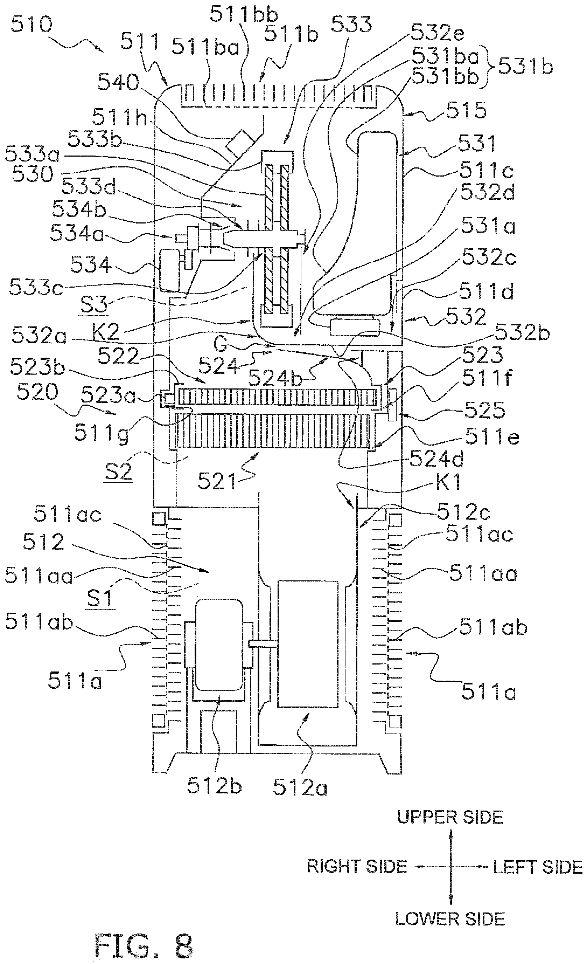

FIG. 7 is a cross-sectional view of the air purifier 510 taken along arrow VII-VII in FIG 6. In other words, FIG. 7 is a view of the interior of the air purifier 510 of FIG. 6 seen from the right side. FIG. 8 is a cross-sectional view of the air purifier 510 taken along arrow VIII-VIII in FIG 6. In other words, FIG 8 is a view of the interior of the air purifier 510 of FIG. 6 seen from the front side.

The air purifier 510 according to the present embodiment has an air purification function and a humidification function (humidity regulation function). The air purifier 510 is configured to be capable of selectively performing the humidification function (configured to be capable of turning on/off the humidification function). Specifically, when a user selects the humidification function (when the humidification function is turned on), both the air purification function and the humidification function of the air purifier 510 are enabled to work. On the other hand, when the user does not select the humidification function (when the humidification function is turned off), only the air purification function of the air purifier 510 is enabled to work. It should be noted that the configuration of turning on/off the functions is not limited to the above, and the air purifier 510 may be configured to constantly enable the air purification function and the humidification function to work. It should be noted that it is easier to reliably achieve comfortableness of the user when the humidification function is configured to be selectively executable.

The air purifier 510 mainly includes a casing 511 (see FIG 6), a blower device 512 (see FIG. 7), an air purification filter 520 (see FIG. 7), a filter case 523 (FIG. 8), a filter detection sensor 525 (FIG 8) and a humidification unit 530 (see FIG 8). The casing 511 accommodates the blower device 512, the air purification filter 520, the filter case 523, the filter detection sensor 525 and the humidification unit 530. The blower device 512 is configured to take air therein from the outside of the casing 511 and feed the taken air to the air purification filter 520 and the humidification unit 530. The air purification filter 520 removes dust and dirt contained in air, and simultaneously, odor components contained in air. The filter case 523 accommodates a deodorization filter 522 of the air purification filter 520 to be described. The filter detection sensor 525 is configured to detect whether or not the deodorization filter 522 of the air purification filter 520 is attached to the casing 511. The humidification unit 530 is configured to vaporize water to be supplied thereto in order to humidify air.

### (2) Detailed Configuration

Main constituent elements of the air purifier 510, including the casing 511, the blower device 512, the air purification filter 520, the filter case 523, the filter detection sensor 525 and the humidification unit 530, will be hereinafter explained in detail.

### (2-1) Casing

The casing 511 accommodates constituent machines and components of the air purifier 510 including the blower device 512, the air purification filter 520, the filter case 523, the filter detection sensor 525, the humidification unit 530 and so forth. The appearance of the casing 511 is made in the shape of a vertically elongated rectangular prism and, in other words, is made in the shape of a rectangular prism elongated in the up-and-down direction. A space for accommodating the constituent machines and components is produced in the interior of the casing 511. As shown in FIG 6, a control panel 513 is mounted to an upper part of a lateral surface (an upper part of the front surface) of the casing 511. The control panel 513 is provided with a variety of switches for operating the air purifier 510 (e.g., a power switch and a function select switch). The control panel 513 is connected to a control device (not shown in the drawings) embedded in the casing 511. A variety of commands, received by the control panel 513, are configured to be transmitted to the control device as signals. The control device is configured to control the action of the air purifier 510 based on the commands received by the control panel 513 and detection results of a variety of sensors including the filter detection sensor 525.

The interior of the casing 511 is mainly divided into three spaces, and thus, a blowing compartment S1, an air purification compartment S2 and a humidification compartment S3 are produced (see FIGS. 7 and 8).

The blower device 512 is disposed in the blowing compartment S1 (see FIGS. 7 and 8). The air purification filter 520 is disposed in the air purification compartment S2 (see FIGS. 7 and 8). The humidification unit 530 is disposed in the humidification compartment S3 (see FIGS. 7 and 8). The blowing compartment S1, the air purification compartment S2 and the humidification compartment S3 are sequentially aligned from bottom to top (see FIGS. 7 and 8). In other words, the blower device 512, the air purification filter 520 and a humidification element 533 (see FIG. 7) of the humidification unit 530 to be described are sequentially disposed from bottom in the casing 511 of the air purifier 510. Here, the humidification unit 530, requiring a maintenance work, is disposed in an upper workable position, and the humidification unit 530 is accessible through an opening 515 (to be described) bored in an upper part of the left surface of the casing 511. Hence, the maintenance work is easily done.

A first opening K1 is provided between the blowing compartment S1 and the air purification compartment S2 (see FIG. 8). The first opening K1 enables air to flow therethrough from the blowing compartment S1 to the air purification compartment S2. Additionally, a second opening K2 is provided between the air purification compartment S2 and the humidification compartment S3 (see FIG. 8). The second opening K2 enables air to flow therethrough from the air purification compartment S2 to the humidification compartment S3.

The areas and positional arrangements of the first opening K1 and the second opening K2 are similar to those of the first opening 311 and the second opening 312 in the air purifier 10 of the first embodiment

In other words, the projected area of the first opening K1 in projecting the first opening K1 onto a horizontal plane is set to fall in a range of 20% to 30% of the horizontal projected area of the air purification compartment S2. The projected area of the second opening K2 in projecting the second opening K2 onto a horizontal plane is set to fall in a range of 40% to 50% of the horizontal projected area of the air purification compartment S2.

Additionally, the first opening K1 and the second opening K2 are displaced in separating directions in a top view (the positional arrangements of the first opening K1 and the second opening K2 are similar to those of the first opening 311 and the second opening 312 in FIGS. 3 and 4, and therefore, the positional arrangements will not be illustrated). Specifically, the first opening K1 is mainly disposed on the left side, whereas the second opening K2 is disposed on the right side. In other words, the first opening K1 is displaced to the left with respect to the center of the air purification filter 520 to be described. On the other hand, the second opening K2 is displaced to the right (oppositely to the first opening K1) with respect to the center of the air purification filter 520 to be described. In the top view, the positional arrangements of the first opening K1 and the second opening K2 are designed for reducing or eliminating the overlapped area between the first opening K1 and the second opening K2 in the top view.

As shown in FIG. 6, the casing 511 is provided with suction ports 511a and a blow-out port 511b. The suction ports 511a and the blow-out port 511b are rectangular openings.

The suction ports 511a are openings for sucking air existing outside the casing 511 into the interior of the casing 511. The suction ports 511a are respectively provided for a lower part of the left surface and a lower part of the right surface of the casing 511 (see FIG. 8). Each suction port 511 a is provided with an inner lattice 511aa and a suction grill 511 ab in order to prevent intrusion of foreign objects and the fingers of the user (see FIG. 8). The inner lattice 511aa is disposed on the inner side of the suction grill 511ab mounted to the outer surface side of the casing 511 (see FIG. 8). Additionally, a pre-filter 511ac is mounted to the inner side of the suction grill 511ab in order to remove large dust and dirt from the air to be taken into the casing 511 (see FIG. 8).

The blow-out port 511b is an opening for blowing out the air that has passed through the interior of the casing 511. In other words, the blow-out port 511b is an air outlet of the casing 511. The air purified by the air purification filter 520 or the air humidified by the humidification unit 530 after air purification by the air purification filter 520 is blown out from the blow-out port 511b. As shown in FIG. 6, the blow-out port 511b is provided on the top surface (ceiling part) of the casing 511. The blow-out port 511b is provided with a metal mesh 511ba and a blow-out grill 511bb in order to prevent intrusion of foreign objects and the fingers of the user (see FIG. 8). The metal mesh 511ba is disposed inside (below) the blow-out grill 511bb mounted to the outer surface side of the casing 511 (see FIG 8).

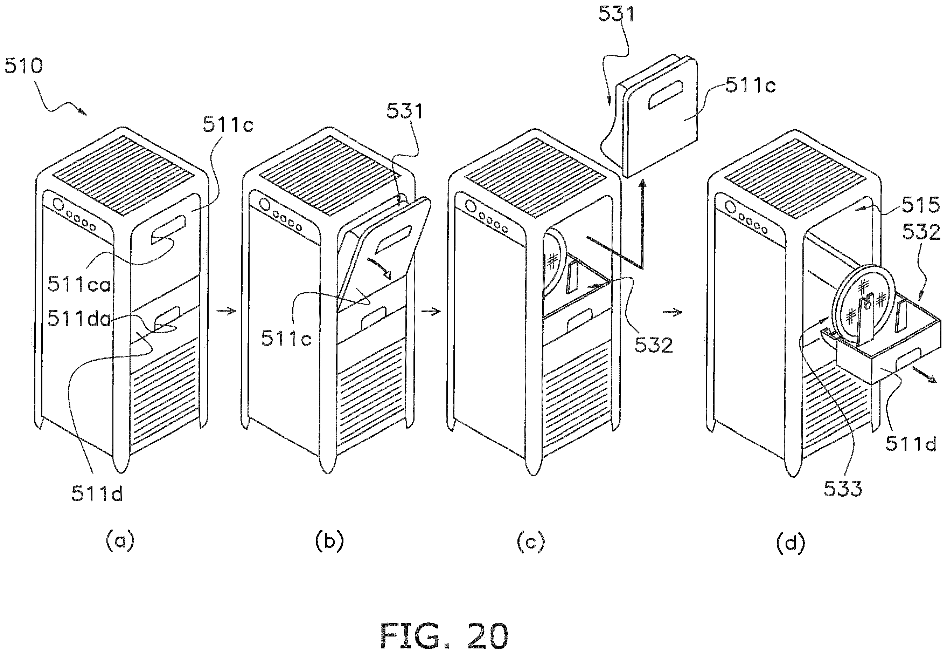

The opening 515 is bored in an upper part of the left surface of the casing 511 (see FIG 8 and FIG 20(d)). The opening 515 is configured to be closable/openable by attaching/detaching a first side plate 511c and a second side plate 511d (see FIG 6). The first side plate 511c is integrated with a humidification tank 531 of the humidification unit 530 to be described (see FIG. 8). The second side plate 511d composes part (left sidewall) of a humidification tray 532 of the humidification unit 530 to be described (see FIG. 8). The first side plate 511c is provided with a pull 511ca to be used in installing/removing the first side plate 511c (i.e., in installing/removing the humidification tank 531) (see FIG. 6). The second side plate 511d is provided with a pull 511da to be used in installing/removing the second side plate 511d (i.e., in installing/removing the humidification tray 532) (see FIG 6).

The opening 515 is an opening for removing constituent elements accommodated in the casing 511 and for installing the constituent elements in the casing 511. Specifically, the opening 515 is an opening to be used for installing/removing (attaching/detaching) the humidification unit 530 and the air purification filter 520 to/from the interior of the casing 511. Here, both of the humidification unit 530 and the air purification filter 520 are configured to be accessible through the single opening 515 not through openings separately provided, i.e., an opening exclusively for a maintenance work of the humidification unit 530 and an opening exclusively for a maintenance work of the air purification filter 520. Therefore, air-tightness of the casing 511 is enhanced, and it is easy to prevent occurrence of air leakage through a gap produced in the opening for a maintenance work. Attachment/detachment of the humidification unit 530 and the air purification filter 520 through the opening 515 will be described below.

A flanged part 511e is provided in the interior of the casing 511 to put thereon a dust collecting filter 521 of the air purification filter 520 to be described (see FIGS. 7 and 8). The flanged part 511e is provided on the inner wall of the casing 511 in the air purification compartment S2. The flanged part 511e is a horizontal support surface for supporting the dust collecting filter 521 from below. The flanged part 511e is disposed below the dust collecting filter 521 of the air purification filter 520 and, in other words, is disposed on the upstream side with respect to the dust collecting filter 521 in the flow direction of air (see FIGS. 7 and 8).

The flanged part 511e is provided over the entire circumference of the inner wall of the casing 511. The flanged part 511e is tightly fitted to a lower surface 521ba (see FIG. 7) of a circumferential edge 521b (see FIG. 11) of the dust collecting filter 521 of the air purification filter 520, and thus, functions as seal means for sealing a gap between the dust collecting filter 521 and the casing 511. It should be noted that a seal member made of resin (not shown in the drawings), for instance, may be disposed between the flanged part 511e and the air purification filter 520 in order to more reliably seal the gap between the dust collecting filter 521 and the casing 511.

A support part 511f is provided in the interior of the casing 511 to put thereon the filter case 523 in which the deodorization filter 522 of the air purification filter 520 to be described is accommodated (see FIG. 8). The support part 511f is disposed on the left surface of the inner wall of the casing 511 in the air purification compartment S2 (see FIG. 8). The support part 511f is disposed in a higher position than the flanged part 511e (see FIG. 8). The support part 511f is a horizontal support surface for supporting from below the filter case 523 in which the deodorization filter 522 is accommodated (see FIG 8).

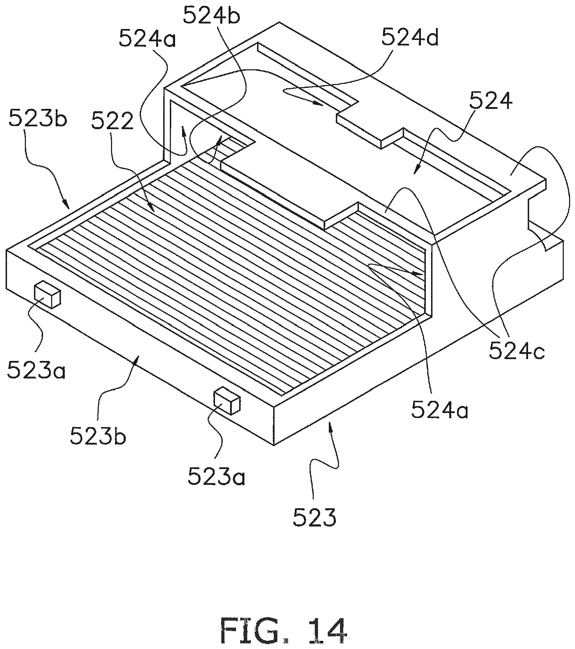

Additionally, recesses 511 g are provided on the inner wall of the casing 511 to be engaged with protrusions 523a provided on the filter case 523 to be described (see FIG 8). Two recesses 511g are separately provided on the right surface of the inner wall of the casing 511. Each recess 511g is recessed so that a corresponding one of two protrusions 523a on the filter case 523 fits therein (see FIG 14). The recesses 511g are provided in higher positions than the flanged part 511e (see FIG. 8). Additionally, the recesses 511g are provided in positions by which the filter case 523 is set approximately horizontal in a condition that the protrusions 523a provided on the right surface of the filter case 523 are engaged with the recesses 511g while the left surface-side part of the filter case 523 is put on the support part 511f. It should be noted that the lower surface of the filter case 523 makes contact with an upper surface 521bb (see FIG. 7) of the circumferential edge 521b (see FIG. 11) of the dust collecting filter 521 to be described in a condition that the filter case 523 is attached to the casing 511 (i.e., a condition that the protrusions 523a are engaged with the recesses 511g while the left surface-side part of the filter case 523 is put on the support part 511f). Attaching the filter case 523 to the casing 511 will be described below.

A distributing plate 511h is provided in the interior of the casing 511 (see FIG. 8). As shown in FIG. 8, the baffle plate 511h is provided in the humidification compartment S3. The baffle plate 511h is provided above the second opening K2. The baffle plate 511h is disposed on the right side of and in adjacent to the humidification element 533 to be described in the humidification compartment S3. The baffle plate 511h has a flat surface with height gradually increasing from the right side to the left side. The baffle plate 511h guides the air, having flown into the humidification compartment S3 through the second opening K2, to flow toward the humidification element 533.

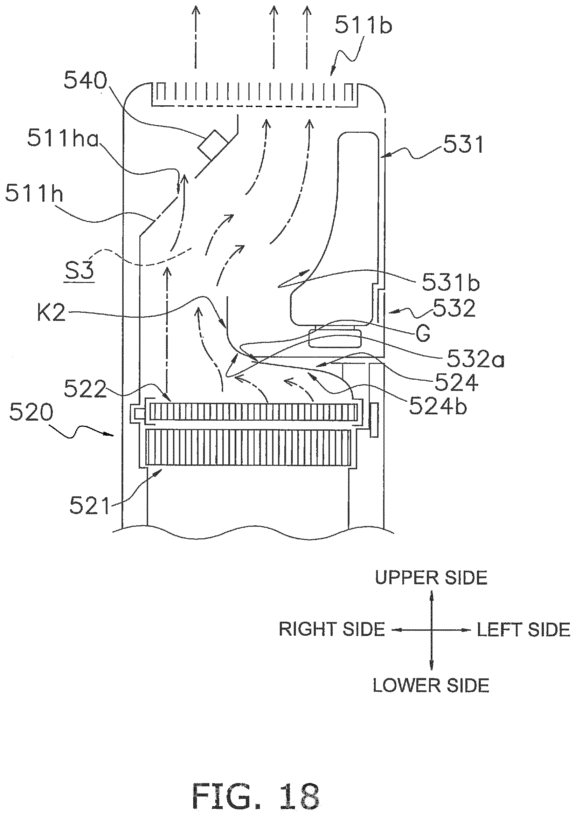

On the other hand, bypass openings 511ha are bored in the baffle plate 511h (see FIG. 18). The bypass openings 511ha are disposed above the second opening K2 (see FIG 18). As depicted with dashed dotted arrows in FIG. 18, the bypass openings 511ha direct part of the air, having passed through the air purification filter 520, from the humidification compartment S3 to the outside of the casing 511 without making it pass through the humidification element 533. Due to the bypass openings 511ha, the airflow rate of the air passing through the air purification filter 520 can be increased in comparison with a configuration that all the air is directed to the humidification element 533 in which relatively large pressure loss is caused. It should be noted that an air ionizer 540 is provided on a surface of the baffle plate 511h, which is disposed on the opposite side of the humidification compartment S3 (see FIG. 8). When an operation of the air ionizer 540 is instructed through the control panel 513, the air ionizer 540 is configured to generate plasma ions. The plasma ions are directed to the outside of the casing 511 by the air blown out to the outside of the casing 511 through the bypass openings 511ha.

### (2-2) Blower Device

The blower device 512 is disposed in the blowing compartment S1 (see FIG. 8). The blower device 512 is a device configured to take the air existing outside the casing 511 into the blowing compartment S1 and blow the taken air to the air purification compartment S2 and the humidification compartment S3. In other words, the blower device 512 is a device configured to blow air to the air purification filter 520 and the humidification unit 530 (the humidification element 533).

The blower device 512 mainly includes a fan 512a, a fan motor 512b and a scroll casing 512c (see FIG 8). The fan 512a is disposed on the left side in the blowing compartment S1, whereas the fan motor 512b is disposed on the right side in the blowing compartment S1 (see FIG. 8). The scroll casing 512c forms an air passage for directing the air blown out from the fan 512a to the first opening K1 located above the fan 512a.

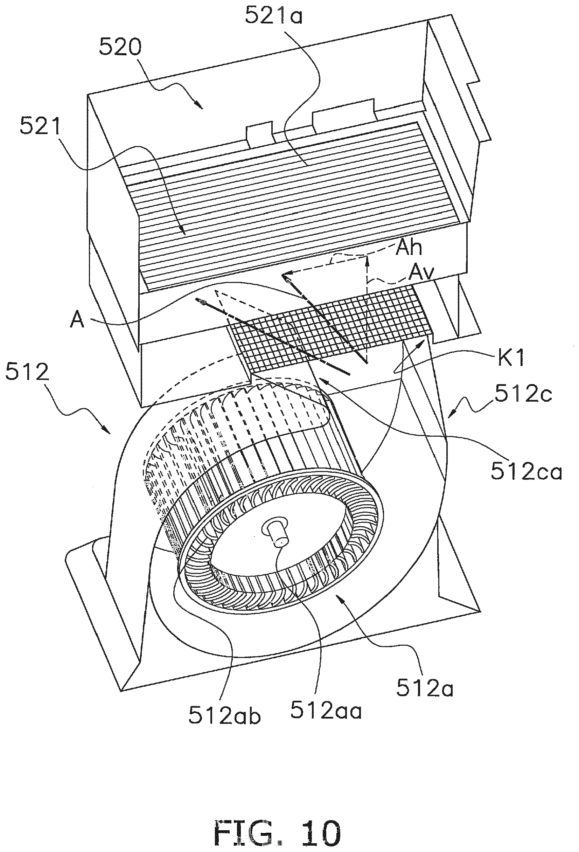

The fan 512a is a sirocco fan in which a stable airflow rate is achieved. The fan 512a includes a hub unit 512aa and a plurality of blades 512ab (see FIG. 10). The blades 512ab are cylindrically aligned on the circumferential edge of the hub unit 512aa. When the fan 512a is driven by the fan motor 512b and thereby the hub unit 512aa and the blades 512ab are rotated, air is sucked into the fan 512a along the direction of the rotational axis of the fan 512a. At this time, air is taken into the blowing compartment S1 from the outside of the casing 511 through the suction ports 511a. In the fan 512a, air is blown out from the blades 512ab in the centrifugal directions. The air, blown out from the blades 512ab in the centrifugal directions, is directed to the first opening K1 through the interior of the scroll casing 512c, and is fed to the air purification compartment S2.

It should be noted that as described above, air is blown out from the blades 512ab in the centrifugal directions, and hence, as depicted with dashed dotted arrows in FIG. 10, air flows not upward but obliquely upward into the dust collecting filter 521 of the air purification filter 520.

### (2-3) Air Purification Filter

The air purification filter 520 purifies the air sucked into the air purifier 510. Specifically, the air purification filter 520 removes dust and dirt contained in air. Additionally, the air purification filter 520 removes causative components of odor.

The air purification filter 520 is disposed in the air purification compartment S2 (see FIG. 8). The air purification filter 520 is disposed above the blower device 512 and below the humidification unit 530 (see FIG. 8). Additionally, the air purification filter 520 is disposed downstream of the blower device 512 and upstream of the humidification unit 530 in the flow direction of air.

The air purification filter 520 mainly includes the dust collecting filter 521 and the deodorization filter 522 (see FIG. 7). The dust collecting filter 521 removes dust and dirt contained in air. The deodorization filter 522 removes causative components of odor contained in air.

The deodorization filter 522 is disposed above the dust collecting filter 521. In other words, the deodorization filter 522 is disposed downstream of the dust collecting filter 521 in the flow direction of air.

The thickness direction of the dust collecting filter 521 and that of the deodorization filter 522 correspond to the up-and-down direction. In other words, the up-and-down direction is defined as the thickness direction of the air purification filter 520. The air passes through the air purification filter 520 mainly in the thickness direction. In other words, the air purification filter 520 (the dust collecting filter 521 and the deodorization filter 522) is disposed to extend in a direction orthogonal to the direction of the air passing therethrough.

### (2-3-1) Dust Collecting Filter

The dust collecting filter 521 is a filter for removing fine dust and dirt that have not been removed by the pre-filters 511ac provided in the suction ports 511a. The dust collecting filter 521 is made in a rectangular shape. The horizontal projected area of a filter part (part causing air to pass therethrough) of the dust collecting filter 521 is designed to be greater than or equal to 80% of that of the air purification compartment S2.

As the dust collecting filter 521, a pleat filter is used that is folded along a plurality of creases 521a and thereby a plurality of "pleats" are formed (see FIG 11). When the dust collecting filter 521 is cut along a plane orthogonal to creases 521a, the cross-section thereof is made in the form of a plurality of continuous peaks and troughs as shown in FIG 12(a). When the installation area of the dust collecting filter 521 is constant, the surface area (air passage area) of the dust collecting filter 521 can be larger in use of the pleat filter than in use of a flat plate filter in which "pleats" are not formed.

It should be noted that the dust collecting filter 521 is put on the flanged part 511e such that the extending direction of the creases 521a of the pleat filter is orthogonal to the extending direction of a tongue 512ca of the scroll casing 512c of the blower device 512, in other words, the direction of the rotational axis of the fan 512a of the blower device 512. Therefore, the extending direction of the creases 521a of the dust collecting filter 521 and the direction of a horizontal component of the air blown out from the scroll casing 512c of the blower device 512 are approximately parallel to each other. This will be herein explained graphically. As shown in FIG. 10, when air is blown out from the scroll casing 512c in the direction of a vector A (obliquely upward direction), the extending direction of the creases 521a of the dust collecting filter 521 and the direction of a horizontal component Ah of the vector A are approximately parallel to each other. It should be noted that the thickness direction of the dust collecting filter 521 corresponds to the direction of a vertical component Av of the vector A.

The following is the reason for installing the dust collecting filter 521 in the aforementioned direction.

It is herein assumed that the dust collecting filter 521 is put on the flanged part 511e such that the extending direction of the creases 521a of the pleat filter and the extending direction of the tongue 512ca of the scroll casing 512c of the blower device 512 are parallel to each other. In this case, the extending direction of the creases 521a and the direction of the horizontal component of the air blown out from the scroll casing 512c are approximately orthogonal to each other. Therefore, as depicted with dashed dotted arrows in FIG 12(b), the air blown out obliquely upward is likely to unevenly pass through the dust collecting filter 521. For example, when this is explained with FIG. 12(b), air is likely to pass through only slopes with height increasing from the left side to the right side in FIG 12(b). Therefore, the entire surface of the dust collecting filter 521 cannot be used for collecting dust.

By contrast, in the air purifier 510 of the present embodiment, the extending direction of the creases 521a and the direction of the horizontal component of the air blown out from the scroll casing 512c are approximately parallel to each other. Hence, as depicted with dashed dotted arrows in FIG 12(a), it is easy to make air evenly flow to the entire surface of the dust collecting filter 521, and structural pressure loss can be inhibited.

It should be noted that when there is a sufficient distance between the fan 512a and the dust collecting filter 521, the aforementioned drawback is relieved and air is likely to upwardly flow into the dust collecting filter 521 even if a type of fan such as a sirocco fan configured to blow out air in the centrifugal directions is used as the fan 512a. Therefore, regardless of the orientation of the dust collecting filter 521, it becomes easy to make air evenly flow to the entire surface of the dust collecting filter 521. It should be noted that for the purpose of compactly producing the air purifier 510, the distance between the fan 512a and the dust collecting filter 521 is preferably short. When the distance between the fan 512a and the dust collecting filter 521 is reduced, the dust collecting filter 521 is preferably disposed in the aforementioned orientation.

As described above, the dust collecting filter 521 of the air purification filter 520 is put on the flanged part 511e formed in the interior of the casing 511. In other words, the dust collecting filter 521 of the air purification filter 520 is held by the flanged part 511e in the casing 511. The flanged part 511e makes contact with the lower surface 521ba of the circumferential edge 521b (see FIG 11) blocking air passage in the dust collecting filter 521 (see FIG 7), and thus functions as seal means for sealing a gap between the air purification filter 520 and the inner wall of the casing 511. The seal means reliably seals between the air purification filter 520 and the casing 511 in feeding air from the blowing compartment S1 to the air purification compartment S2. The flanged part 511e is disposed upstream of the air purification filter 520 in the flow direction of air. In other words, the seal means is disposed upstream of the air purification filter 520 in the detachment direction of the air purification filter 520. Attachment/detachment of the dust collecting filter 521 to the casing 511 will be described below.

### (2-3-2) Deodorization Filter

The deodorization filter 522 is a filter for removing causative components of odor contained in air. The deodorization filter 522 is a filter made in a rectangular shape. The horizontal projected area of a filter part (part causing air to pass therethrough) of the deodorization filter 522 is designed to be greater than or equal to 80% of that of the air purification compartment S2.

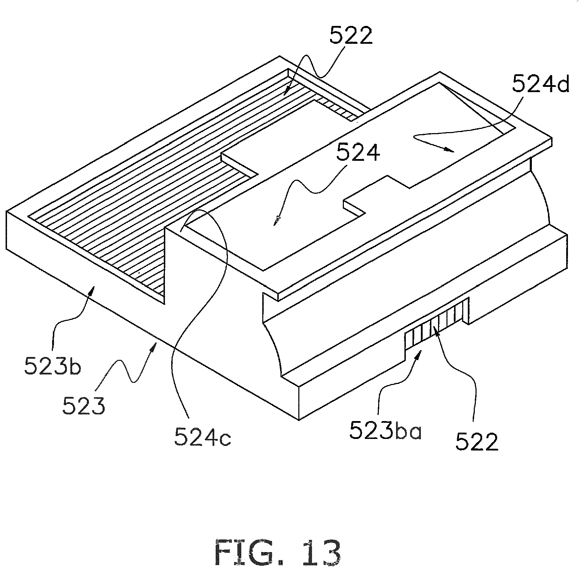

As shown in FIGS. 13 and 14, the deodorization filter 522 is accommodated in the filter case 523 to be described. The deodorization filter 522 is configured to be installable to and removable from the filter case 523. A condition of the filter case 523 attached to the casing 511 and attachment/detachment of the deodorization filter 522 (the filter case 523) to/from the casing 511 will be described below. The filter detection sensor 525 for checking existence/non-existence of the deodorization filter 522 will be also described below.

### (2-4) Filter Case

The filter case 523 accommodates the air purification filter 520. Specifically, the filter case 523 accommodates the deodorization filter 522 that is part of the air purification filter 520 (see FIG. 13).

The filter case 523 is a ring-shaped member made in a rectangular shape, and accommodates the deodorization filter 522 in the interior thereof. The filter case 523 includes a sidewall part 523b (see FIGS. 7 and 8) having a roughly C-shaped cross-section, and the sidewall part 523b surrounds and holds the circumferential edge of the accommodated deodorization filter 522 from the top, bottom and lateral (outer peripheral) sides. In other words, the inner peripheral side of the sidewall part 523b is opened. The entire circumference of the deodorization filter 522 is surrounded by the sidewall part 523b. The top and bottom surfaces of the filter case 523 are largely opened, and the air, having passed through the dust collecting filter 521, passes through the deodorization filter 522 accommodated in the filter case 523 from below and upwardly blows out from the deodorization filter 522.

The protrusions 523a are provided on the right surface of the sidewall part 523b of the filter case 523, and are configured to be engaged with the recesses 511g provided on the inner wall of the casing 511. Two protrusions 523a are separately provided on the right surface of the sidewall part 523b. It should be noted that the number of the protrusions 523a is exemplary only and may be suitably set without being limited to the above. The number of the recesses 511g provided on the inner wall of the casing 511 may be also suitably set in accordance with the number of the protrusions 523a. Each protrusion 523a is formed to fit into corresponding one of the two recesses 511g (see FIG 11) separately provided on the casing 511.

In a condition that the filter case 523 is attached to the casing 511, the protrusions 523a are engaged with the recesses 511g, and the left surface-side part of the filter case 523 is put on the support part 511f provided in the interior of the casing 511. In other words, in the condition that the filter case 523 is attached to the casing 511, the filter case 523 is supported from below by the support part 511f and the recesses 511g which fits to the protrusions 523a. In the condition that the filter case 523 is attached to the casing 511, the deodorization filter 522 is attached to the casing 511 in a horizontally oriented condition, in other words, in a condition that the thickness direction of the deodorization filter 522 and the up-and-down direction correspond to each other. Attachment/detachment of the filter case 523 to/from the casing 511 will be described below.

A cutout 523ba is formed in the middle of the left surface of the sidewall part 523b of the filter case 523 (see FIG. 13). In the sidewall part 523b where the cutout 523ba is formed, an upper part (the top surface and an upper part of the lateral surface) is left uncut (see FIG 13). In a condition that the deodorization filter 522 is accommodated in the filter case 523, the deodorization filter 522 is configured to be exposed through the cutout 523ba.

It should be noted that the filter case 523 includes an airflow guide 524 to be disposed above the air purification filter. The airflow guide 524 is integrated with the filter case 523.

The airflow guide 524 is provided above the left part of the filter case 523 (see FIG. 8). The airflow guide 524 is provided above the first opening K1 formed in the casing 511. The airflow guide 524 includes vertical surfaces 524a upwardly extending from the left regions of the front and rear surfaces of the sidewall part 523b and a first guide surface 524b (see FIG 14) extending obliquely upward to the right from the left surface of the sidewall part 523b. The first guide surface 524b is a curved surface for directing the air, having passed through the deodorization filter 522 of the air purification filter 520, to the humidification element 533 of the humidification unit 530 to be described. With the first guide surface 524b made in the form of a smoothly curved surface, the air having passed through the deodorization filter 522 can be directed to the second opening K2 and further to the humidification element 533 while preventing the elevation in pressure loss. The vertical surfaces 524a of the airflow guide 524 prevent a situation that the air, having passed through the deodorization filter 522, upwardly flows through gaps that could be produced on the front and rear sides of the first guide surface 524b of the airflow guide 524 and then flows out from the casing 511 through gaps between the casing 511 and the humidification tray 532 to be described. The vertical surfaces 524a and the first guide surface 524b mainly direct the air, having passed through the left part of the deodorization filter 522, to the humidification element 533.

A recess 524d, made in a downwardly recessed shape, is provided on the upper part of the airflow guide 524 (see FIG. 14). The recess 524d is made in a shape not only recessed downward but also recessed to a position below the left part of a top surface 524c of the airflow guide 524 (see FIG. 14). The recess 524d functions as a pull to be held by a user in attaching/detaching the filter case 523.

In a condition that the air purification filter 520 and the humidification unit 530 are attached to the casing 511, the top surface 524c of the airflow guide 524 (see FIGS. 13 and 14) is configured to make contact with a bottom surface 532b of the humidification tray 532 of the humidification unit 530 to be described (see FIG. 8). Therefore, even if air is blown from the blower device 512 and a force to float (i.e., upwardly move) acts to the air purification filter 520, the humidification tray 532 restricts upward movement of the air purification filter 520. In other words, the bottom surface 532b of the humidification tray 532 functions as a movement restricting part for restricting upward movement of the air purification filter 520.

### (2-5) Filter Detection Sensor

The filter detection sensor 525 (see FIG. 8) is a sensor configured to detect whether or not the air purification filter 520 is attached to the casing 511. Specifically, the filter detection sensor 525 is a sensor for detecting whether or not the deodorization filter 522 of the air purification filter 520 is attached to the casing 511.

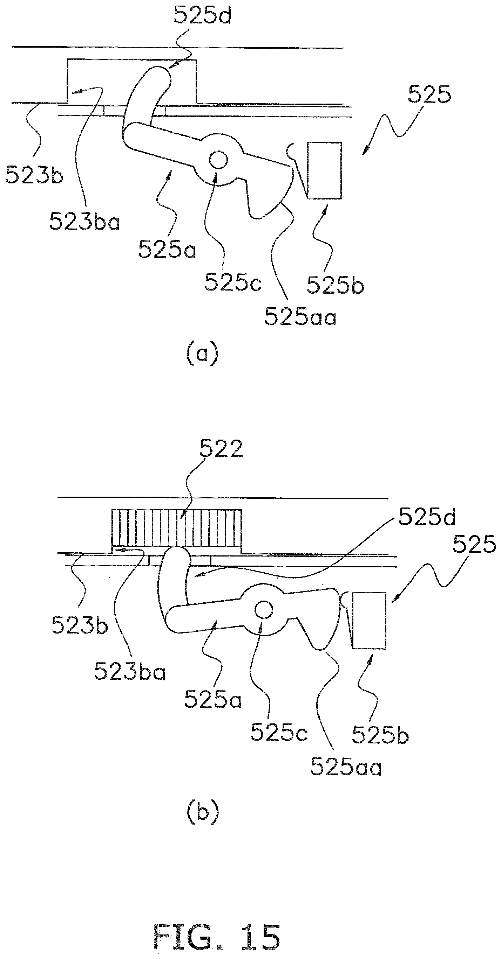

The filter detection sensor 525 mainly includes a detection lever 525a and a detection switch 525b (see FIGS. 15(a) and 15(b)). The detection lever 525a is configured to be rotatable about a rotation axis 525c (see FIGS. 15(a) and 15(b)). The detection lever 525a includes a contact arm 525d on one end thereof. The contact arm 525d extends in a direction intersecting with the extending direction of the detection lever 525a and is configured to make contact with the deodorization filter 522. A detection switch contact part 525aa is provided on the other end of the detection lever 525a (i.e., an end disposed on the opposite side of the end, on which the contact arm 525d is provided, through the rotation shaft 525c) and is configured to make contact with the detection switch 525b. It should be noted that the contact arm 525d herein extends in a circular-arc shape from the detection lever 525a, but the shape of the contact arm 525d is not limited to this. The contact arm 525d may be made in a shape extending perpendicularly to the extending direction of the detection lever 525a.

A torsion spring (not shown in the drawings) is configured to apply a force to the detection lever 525a in one direction about the rotation shaft 525c (i.e., a direction that the detection switch contact part 525aa is separated away from the detection switch 525b). In a condition that the contact arm 525d is not making contact with the deodorization filter 522 (i.e., a condition that the deodorization filter 522 is not accommodated in the filter case 523), as shown in FIG. 15(a), the contact arm 525d protrudes into the filter case 523 while passing through the cutout 523ba of the filter case 523. In this condition, the detection switch contact part 525aa of the detection lever 525a does not make contact with the detection switch 525b. Therefore, in the condition that the deodorization filter 522 is not accommodated in the filter case 523, a signal configured to be transmitted in existence of the deodorization filter 522 is not transmitted from the filter detection sensor 525 to the control device (not shown in the drawings) of the air purifier 510. In the condition described above, the control device determines that the deodorization filter 522 is not attached to the air purifier 510, and is configured to perform necessary controls such as stopping the operation of the air purifier 510, issuing an alert indicating that the deodorization filter 522 is not attached, and so forth. It should be noted that although not illustrated and explained herein, similarly in a condition that not only the deodorization filter 522 but also the filter case 523 is not attached to the casing 511, the detection switch contact part 525aa does not make contact with the detection switch 525b, and the signal configured to be transmitted in existence of the deodorization filter 522 is not transmitted to the control device.

On the other hand, in the condition that the deodorization filter 522 is accommodated in the filter case 523, the contact arm 525d makes contact with the deodorization filter 522 through the cutout 523ba of the sidewall part 523b of the filter case 523. Then, the detection lever 525a is rotated against the force of the torsion spring (not shown in the drawings) in a direction that the detection switch contact part 525aa of the detection lever 525a makes contact with the detection switch 525b. When the detection switch contact part 525aa makes contact with the detection switch 525b, the detection switch 525b is turned on, and the signal configured to be transmitted in existence of the deodorization filter 522 is transmitted from the filter detection sensor 525 to the control device of the air purifier 510. When the control device determines that the deodorization filter 522 is attached to the air purifier 510, the control device is configured to allow the operation of the air purifier 510 unless there are other malfunctions.

It should be noted that the present air purifier 510 is only provided with the filter detection sensor 525 for detecting existence/non-existence of the deodorization filter 522 as sensors for detecting existence/non-existence of filters. However, the configuration of providing sensors is not limited to this. For example, a sensor utilizing a principle similar to that of the filter detection sensor 525 or another detection principle may be provided for detecting existence/non-existence of the dust collecting filter 521. It should be noted that as described below, in the present air purifier 510, when the dust collecting filter 521 does not exist, it becomes difficult to attach the filter case 523, in which the deodorization filter 522 is accommodated, to the air purifier 510. Hence, only by detecting existence/non-existence of the deodorization filter 522, it is also possible to detect the condition that the dust collecting filter 521 is not attached.

### (2-6) Humidification Unit

The humidification unit 530 is configured to vaporize water supplied thereto in order to humidify air. Specifically, the humidification element 533 of the humidification unit 530 is particularly configured to vaporize water supplied thereto in order to humidify air. The humidification unit 530 is disposed downstream of the blower device 512 and the air purification filter 520 in the flow direction of air. In other words, the humidification unit 530 is configured to humidify the air that has been purified by the air purification filter 520. The humidification unit 530 is a humidification unit of a rotary type. In the humidification unit 530, the circumferential edge of the humidification element 533 is configured to be rotated to pass through water retained in the humidification tray 532 to be described, and then, water absorbed by the humidification element 533 is vaporized to humidify the air.

The humidification unit 530 is disposed in the humidification compartment S3 within the casing 511 (see FIG. 8). In other words, the humidification unit 530 is disposed in an upper position within the casing 511 (see FIG. 8). The humidification tank 531, the humidification tray 532 and the humidification element 533 of the humidification unit 530 to be described are configured to be installable to and removable from the casing 511. When the humidification tank 531, the humidification tray 532 and the humidification element 533 are removed through the opening 515 (see FIG 8) of the casing 511, a large space is produced in an upper position within the casing 511. Specifically, when the humidification unit 530 is removed from the casing 511, a space in which the air purification filter 520 is movable is produced in the humidification compartment S3. In other words, when the humidification unit 530 is detached from the casing 511, a space serving as a moving path of the air purification filter 520 in detaching the air purification filter 520 is produced above the air purification compartment S2. It should be noted that attachment/detachment of the air purification filter 520 will be described.

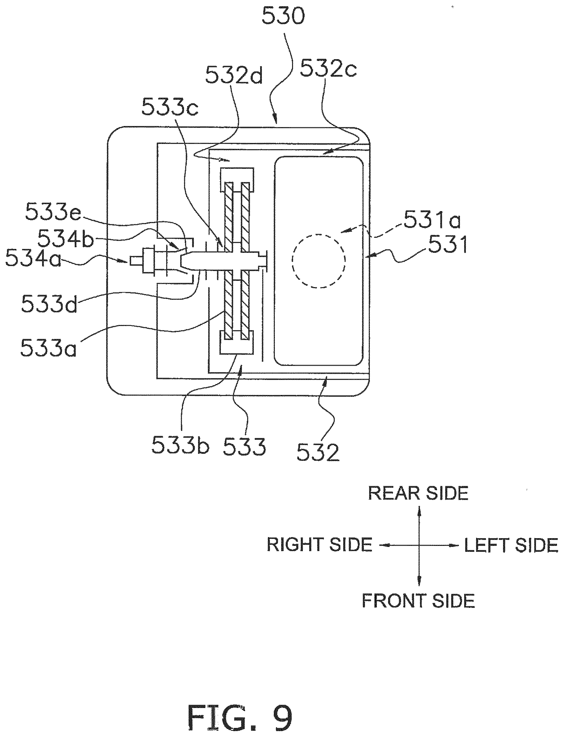

As shown in FIGS. 8 and 9, the humidification unit 530 mainly includes the humidification tank 531, the humidification tray 532 and the humidification element 533. Additionally, the humidification unit 530 includes a humidification motor 534 for rotating the humidification element 533 (see FIG. 8). The humidification motor 534 is installed in the interior of the casing 511. On the other hand, the humidification tank 531, the humidification tray 532 and the humidification element 533 are configured to be detachable from the casing 511.

### (2-6-1) Humidification Tank

The humidification tank 531 is configured to store water to be supplied to the humidification element 533. The humidification tank 531 is integrated with the first side plate 511c of the casing 511. Therefore, the humidification tank 531 is configured to be installed/removed to/from the humidification unit 530 (the humidification tray 532) by detaching the first side plate 511c from the casing 511 (see FIG 19(b)).

The humidification tank 531 is disposed on the left part of the humidification tray 532. The humidification tray 532 is disposed in the left part of the casing 511, and hence, the humidification tank 531 is disposed on the left part of the casing 511.

In the present air purifier 510, the fan motor 512b of the blower device 512, which is a heavy object as described above, is disposed in the right part of the casing 511 (see FIG. 8). On the other hand, the humidification tank 531, which is a heavy object, is disposed in the left part of the casing 511 (see FIG 8). In other words, the fan motor 512b is displaced to one lateral side (i.e., rightward) with respect to the center of the air purification filter 520, whereas the humidification tank 531 is displaced oppositely to the aforementioned one side (i.e., leftward) with respect to the center of the air purification filter 520. Thus, the heavy fan motor 512b and the heavy humidification tank 531 are separated away from each other in opposite directions without being disposed only on the same side in a top view. Hence, the present air purifier 510 is well balanced in weight.

The humidification tank 531 has a water filling port 531a. A water feed valve (not shown in the drawings) is attached to the water filling port 531a. The water feed valve has a general structure in which a valve element is pushed onto a valve port by a spring. Therefore, detailed explanation of the water feed valve will not be provided herein.

In use, the humidification tank 531 is disposed on a tank receiver 532c (to be described) that is located on the left part of the humidification tray 532. Specifically, the humidification tank 531 is mounted to the tank receiver 532c of the humidification tray 532, with the water filling port 531a being directed downward. When the humidification tank 531 is mounted to the tank receiver 532c, a push pin (not shown in the drawings) mounted to the humidification tray 532 is pushed onto the water feed valve of the water filling port 531a directed downward. Then, by the weight of the humidification tank 531, the push pin pushes the water feed valve and a valve port is pushed and opened. Accordingly, the water filling port 531a is opened. As a result, water is supplied to the humidification tray 532 to be described, and is retained to a predetermined level in the humidification tray 532 so as to immerse therein the humidification element 533.

When seen from the right side, the humidification tank 531 is made in an approximately rectangular shape (see FIG 7). When seen from the front side, the left lateral part of the humidification tank 531 is made in the shape of a flat surface extending in an approximately vertical direction (see FIG. 8). Additionally, when seen from the front side, the right lateral part of the humidification tank 531 is made in the shape of a curved surface (see FIG. 8). A right lateral surface 531b (see FIG 8) of the humidification tank 531, made in the shape of a curved surface, functions as a guide surface for directing the air, having passed through the humidification element 533, to the blow-out port 511b. Specifically, a lower part (a right lateral surface lower part 531ba) of the right lateral surface 531b is a curved surface recessed in a down left direction. The right lateral surface lower part 531ba is a curved surface with height gradually increasing toward the left side (see FIG 8). The right lateral surface lower part 531ba is a curved surface with slope changing to gradually approach vertical toward the left side (see FIG 8). An upper part of the right lateral surface 531b (a right lateral surface upper part 531bb) is a vertical surface smoothly continuing to the right lateral surface lower part 531ba (see FIG. 8). With the right lateral surface 531b made in the aforementioned shape, the air, having passed through the humidification element 533 obliquely upward to the left, is directed to the blow-out port 511b bored in the upper part of the casing 511 without causing a large pressure loss.

### (2-6-2) Humidification Tray

The humidification tray 532 is configured to receive water supplied from the humidification tank 531 and temporarily retain it. Specifically, the humidification tray 532 is configured to retain water to be supplied to the humidification element 533 to be described. The second side plate 511d of the casing 511 also functions as the left sidewall of the humidification tray 532.

The humidification tray 532 is disposed below the humidification element 533 (see FIG 8). As shown in FIG 8, when seen from a lateral side (front side in FIG 8), the humidification tray 532 is made in an approximately U shape. The humidification tray 532 is disposed in the left part of the casing 511 (see FIG 8). The second opening K2 is produced between the right sidewall of the humidification tray 532 and the inner wall of the casing 511 (see FIG. 8).

The humidification tray 532 includes a second guide surface 532a having a curved shape in the lower edge of the right part of the humidification tray 532. The second guide surface 532a directs the air, having passed through the air purification filter 520, to the humidification element 533 (see FIG 8). In other words, the humidification tray 532 includes the second guide surface 532a having a curved shape on the lower edge thereof adjacent to the second opening K2. The second guide surface 532a directs the air, having passed through the air purification filter 520, to the humidification element 533 (FIG. 8). Specifically, the lower edge of the right part of the humidification tray 532 is processed with curved surface machining.

The aforementioned first guide surface 524b of the airflow guide 524 and the second guide surface 532a form a continuous curve through a slight gap G produced between the airflow guide 524 and the humidification tray 532. It should be noted that the bottom surface 532b of the humidification tray 532 functions as the movement restricting part for restricting upward movement of the air purification filter 520 in a condition that the humidification unit 530 and the air purification filter 520 are attached to the interior of the casing 511. In other words, the bottom surface 532b of the humidification tray 532 as the movement restricting part restricts upward movement of the air purification filter 520 by making contact with the top surface 524c of the airflow guide 524. Therefore, the gap produced between the airflow guide 524 and the humidification tray 532 is quite narrow. Thus, the air, which has passed through the deodorization filter 522 and then been guided by the first guide surface 524b, is mostly directed to the second opening K2 and is then directed to the humidification element 533. In other words, it does not happen that most of the air guided by the first guide surface 524b flows through the gap between the airflow guide 524 and the humidification tray 532, and then, flows out from the casing 511 through gaps between the casing 511 and the first and second side plates 511c and 511d, and so forth.

The humidification tray 532 includes the tank receiver 532c in the left part thereof in order to receive the humidification tank 531 (see FIG. 9). The humidification tank 531 is disposed in the tank receiver 532c, with the water filling port 531a being directed downward (see FIG. 8). The water feed valve of the water filling port 531a of the humidification tank 531 to be mounted to the tank receiver 532c is pushed onto the push pin (not shown in the drawings) mounted to the tank receiver 532c. Then, by the weight of the humidification tank 531, the push pin pushes the water feed valve and the valve port is pushed and opened. Accordingly, the water filling port 531a is opened, and water is supplied to the humidification tray 532.

The water supplied from the humidification tank 531 is supplied to a water supply part 532d that is located in the right part of the humidification tray 532 (see FIG. 9). The water supply part 532d is disposed below the humidification element 533 (see FIG 8). The water supply part 532d is a part for supplying the water, supplied to the tank receiver 532c from the humidification tank 531, to the humidification element 533.

The water supply part 532d and the tank receiver 532c are divided by a partition (not shown in the drawings). The partition has a cutout, and the water supplied to the tank receiver 532c from the humidification tank 531 is fed to the water supply part 532d through the cutout. In other words, the water, supplied to the tank receiver 532c disposed on the same side as the left surface of the casing 511, is then fed to the water supply part 532d faced to the right surface of the casing 511. Water is retained to a predetermined level in the water supply part 532d in order to immerse therein the humidification element 533. When water exists in the humidification tank 531, the level of water in the water supply part 532d is automatically regulated to keep at the predetermined level.

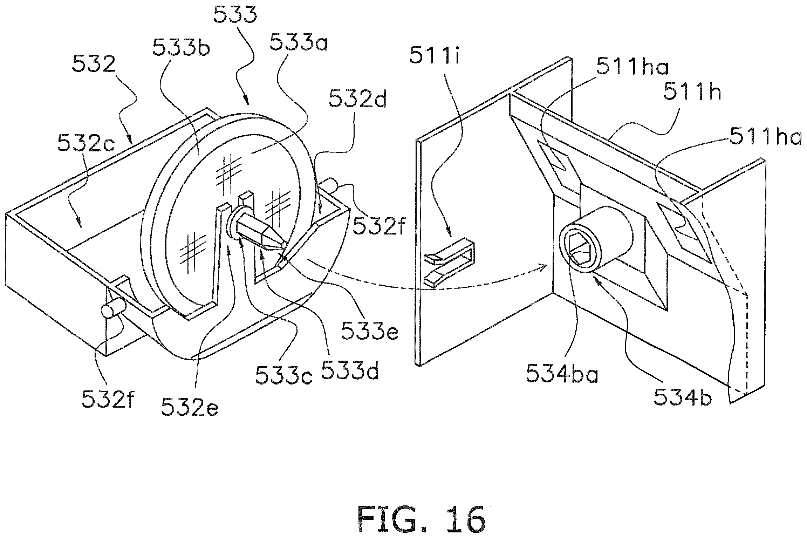

The humidification tray 532 includes a shaft receiver 532e (see FIG. 16). The shaft receiver 532e supports a rotor rotating shaft 533c of the humidification element 533 to be described (see FIG 16) such that the rotor rotating shaft 533c is rotatable. It should be noted that the shaft receiver 532e is constructed to enable the rotor rotating shaft 533c to be installed/removed thereto/therefrom such that the humidification element 533 is detachable in a maintenance work and so forth. Specifically, the shaft receiver 532e is upwardly opened in a U-shape, and is thus constructed to enable the rotor rotating shaft 533c to be attached/detached thereto/therefrom through the U-shaped opening (see FIG. 16).

A single guide pin 532f is provided for each of the front and rear lateral surfaces of the water supply part 532d of the humidification tray 532 (see FIG. 16). In attaching the humidification tray 532 to the casing 511, the humidification tray 532 is pushed into the interior of the casing 511 such that the guide pins 532f are fitted into C-shaped guides 511i (see FIG 16) provided on the inner walls of the front and rear surfaces of the casing 511 through the openings of the C-shaped guides 511i. Accordingly, the humidification tray 532 is easily set in a predetermined position.

### (2-6-3) Humidification Element

The humidification element 533 is configured to vaporize water supplied thereto in order to humidify air. Specifically, the humidification element 533 is configured to receive supply of water retained in the humidification tray 532 and vaporize the supplied water in order to humidify air.

The humidification element 533 is disposed in the right side on the humidification tray 532. Specifically, the humidification element 533 is disposed above the water supply part 532d provided in the right side of the humidification tray 532.

The humidification element 533 is made in a circular shape. The humidification element 533 mainly includes a humidification filter 533a, a humidification rotor 533b and a rotor rotating shaft 533c (see FIG. 16).

The humidification filter 533a is a vaporization material made of non-woven fabric. The humidification filter 533a is made in a circular shape. The humidification filter 533a absorbs water supplied thereto from the humidification tray 532, and vaporizes the absorbed water in order to humidify air.

The humidification rotor 533b is a frame for holding the humidification filter 533a by enclosing the outer peripheral edge of the humidification filter 533a. The humidification rotor 533b is coupled to the rotor rotating shaft 533c disposed in the center thereof.

One end (left end) of the rotor rotating shaft 533c is rotatably supported by the shaft receiver 532e provided on the humidification tray 532. The other end (right end) of the rotor rotating shaft 533c is provided with a coupling part 533d made in a hexagonal shape. Additionally, the right end of the rotor rotating shaft 533c is provided with a tip part 533e that continues to the coupling part 533d and is tapered toward the end thereof.

In attaching the humidification tray 532 to the casing 511, the rotor rotating shaft 533c is inserted into a hole 534ba of a coupling receiver 534b of the humidification motor 534 to be described from the tip part 533e side (see FIG. 16). The rotor rotating shaft 533c is inserted into the hole 534ba until the coupling part 533d reaches the coupling receiver 534b, and thus, the coupling part 533d and the coupling receiver 534b are coupled to each other. Specifically, the hole 534ba of the coupling receiver 534b is a hexagonal hole having approximately the same contour as the coupling part 533d. By inserting the coupling part 533d into the hole 534ba bored in the coupling receiver 534b, the coupling part 533d is fitted to the coupling receiver 534b. In this way, the coupling part 533d and the coupling receiver 534b are coupled to each other. By coupling the coupling receiver 534b and the coupling part 533d to each other, the rotor rotating shaft 533c and a drive shaft 534a of the humidification motor 534 to be described are coupled to each other. When the humidification motor 534 is driven and the drive shaft 534a is thereby rotated, the rotor rotating shaft 533c is rotated accordingly. The coupling part 533d and the hole 534ba of the coupling receiver 534b are herein made in hexagonal shapes. Hence, when the drive shaft 534a is rotated, the coupling receiver 534b is rotated without idling with respect to the coupling part 533d.

The rotor rotating shaft 533c is herein provided with the tip part 533e made in a shape tapering toward the end thereof. Hence, the tip part 533e functions as a guide, and makes it easy to insert the rotor rotating shaft 533c into the hole 534ba of the coupling receiver 534b.

When a driving torque is transmitted from the humidification motor 534 to be described to the rotor rotating shaft 533c and thereby the humidification element 533 is rotated, the circumferential edge of the humidification filter 533a of the humidification element 533 passes through the water retained in the water supply part 532d of the humidification tray 532. When the circumferential edge of the humidification filter 533a passes through the water retained in the water supply part 532d of the humidification tray 532, the humidification filter 533a receives supply of water from the water supply part 532d. When the humidification element 533 is rotated, the air that has been blown by the blower device 512 (the air that has passed through the air purification filter 520) is supplied to a part of the humidification filter 533a moved above the water supply part 532d. When the air passes through the humidification filter 533a, the water absorbed in the humidification filter 533a vaporizes, and thus, the air is humidified.

### (2-6-4) Humidification Motor

The humidification motor 534 is a motor for rotary driving the humidification element 533.

The humidification motor 534 is installed in the interior of the casing 511. Specifically, the humidification motor 534 is fixed to the casing 511 in the right side of the humidification unit 530.

The humidification motor 534 is configured to transmit a driving force to the drive shaft 534a (see FIG. 8). When the humidification motor 534 is rotated, the drive shaft 534a is configured to be rotated accordingly.

The drive shaft 534a includes the coupling receiver 534b in the left side thereof (see FIG 8). The hole 534ba made in a hexagonal shape (see FIG 16) is formed on the coupling receiver 534b. The shape of the hole 534ba is approximately the same as the cross-sectional shape of the rotor rotating shaft 533c. It should be noted that the hole 534ba is made in a hexagonal shape slightly larger than the cross-sectional shape of the coupling part 533d such that the coupling part 533d of the rotor rotating shaft 533c can be inserted into the hole 534ba. As described above, the drive shaft 534a and the rotor rotating shaft 533c are coupled to each other by inserting the coupling part 533d of the rotor rotating shaft 533c into the hole 534ba.

### (3) Action of Air Purifier and Flow of Air

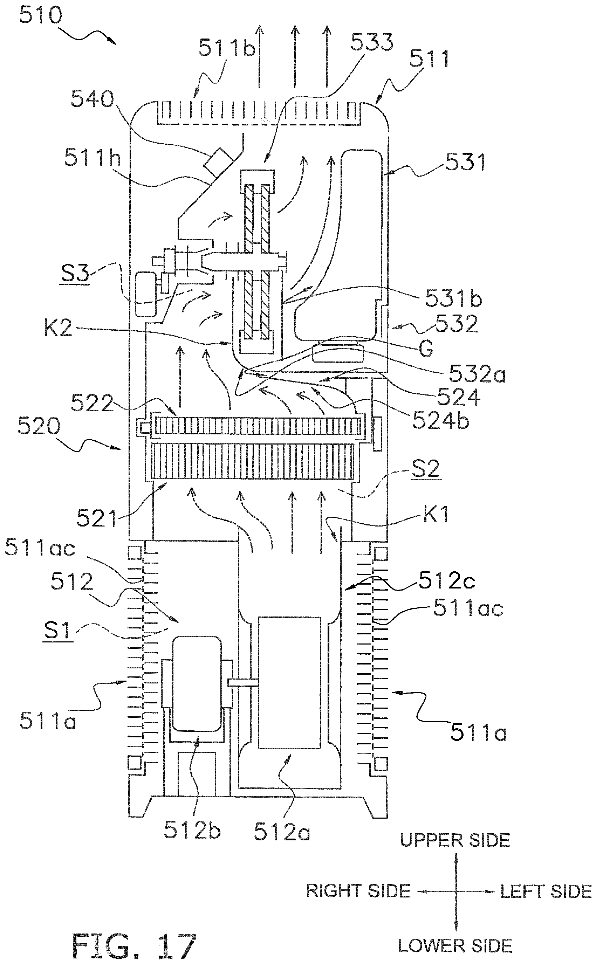

An action of the air purifier 510 and a flow of air to be generated with the action of the air purifier 510 will be hereinafter explained with FIGS. 17 and 18. FIGS. 17 and 18 depict the flow of air with dashed dotted arrows.

When the air purifier 510 is powered on, the fan motor 512b of the blower device 512 rotates the fan 512a. While rotating, the fan 512a blows out air in the centrifugal directions. Hence, pressure decreases in the surroundings of the rotational axis of the fan 512a. Accordingly, air is sucked into a fan suction port located in the center of the fan 512a. As a result, the flow of air, directed from the suction ports 511a to the fan suction port located in the center of the fan 512a, is generated. In other words, when the fan 512a is rotated, air is sucked into the blowing compartment S1 from the suction ports 511a bored in the lower part of the left surface and that of the right surface. At this time, relatively large dust and dirt are removed by the pre-filters 511ac respectively disposed in the suction ports 511a.

The air is blown out from the plural vanes 512ab (see FIG 10) of the fan 512a of the blower device 512 in the centrifugal directions. The air blown out in the centrifugal directions is directed to the first opening K1 by the scroll casing 512c, and is then fed into the air purification compartment S2 through the first opening K1 (see FIG 17).

The air fed to the air purification compartment S2 firstly passes through the dust collecting filter 521 disposed on the upstream side in the flow direction of air in the air purification filter 520. The dust collecting filter 521 removes dust and dirt contained in air.

It should be noted that the flow of air passing through the dust collecting filter 521 includes not only a vertically upward component but also a horizontal component However, the extending direction of the creases 521a of the dust collecting filter 521 of a pleat type and the extending direction of the tongue 512ca of the scroll casing 512c (the direction of the rotational axis of the fan 512a) are herein orthogonal to each other. Hence, it is possible to make air evenly flow to the entire surface of the dust collecting filter 521.

The air, having passed through the dust collecting filter 521, further passes through the deodorization filter 522 disposed on the downstream side in the flow direction of air. Causative components of odor are removed by the deodorization filter 522.

It should be noted that when air passes through the air purification filter 520 (the dust collecting filter 521 and the deodorization filter 522), static pressure acts on the entire surface of the air purification filter 520, and the air passes through the air purification filter 520. At this time, air increases in speed in flowing through the air purification filter 520 at a part opposed to the first opening K1. However, the first opening K1 and the second opening K2 are herein displaced from each other in a top view and an overlapped part between the first and second openings K1 and K2 is sufficiently small. Hence, as also explained in the first embodiment, air is likely to evenly pass through the entire surface of the air purification filter 520, and it is possible to inhibit occurrence of a part through which air does not pass or in which the speed of air is extremely low.

The air, having passed through the air purification filter 520, then passes through the second opening K2, and is fed to the humidification compartment S3. At this time, in particular, the air, having passed through the left part of the deodorization filter 522, is smoothly directed to the second opening K2 by the first guide surface 524b of the airflow guide 524 and the second guide surface 532a formed on the lower part of the humidification tray 532 (see FIG 17). It should be noted that as described above, a contact is established between the top surface 524c of the airflow guide 524 and the bottom surface 532b of the humidification tray 532. Hence, a gap is hardly produced between the airflow guide 524 and the humidification tray 532, and it does not happen that air passes through this gap and then flows out from the casing 511 through gaps between the opening of the casing 511 and the first and second side plates 511c and 511d. The first guide surface 524b and the second guide surface 532a form a continuous curve through the slight gap G produced therebetween, and air is smoothly directed to the second opening K2 along this curve (see FIG 17).

The air, having been fed to the humidification compartment S3, flows upward and is directed to the humidification element 533 by the baffle plate 511h provided in the upper part of the casing 511. It should be noted that two bypass openings 511ha are separately bored in the baffle plate 511h (see FIG 18). Hence, part of the air (e.g., 20% of the air that has flown into the humidification compartment S3) passes through the bypass openings 511ha, and is then directed to the outside of the casing 511 without passing through the humidification element 533 (see FIG 18). The air ionizer 540 is disposed on the surface of the baffle plate 511h, which is disposed on the opposite side of the humidification compartment S3. Plasma ions generated by the air ionizer 540 are carried to the outside of the casing 511 together with the air that has passed through the bypass opening 511ha.

When the humidification function of the air purifier 510 is activated, the humidification element 533 is rotated by the humidification motor 534. The humidification element 533 is disposed such that the lower part thereof is immersed into the water in the water supply part 532d of the humidification tray 532. Hence, when the humidification element 533 is rotated, a part of the humidification filter 533a to be immersed anew into the water absorbs the water in the humidification tray 532. The air passing through the humidification element 533 promotes vaporization of the water that has soaked into the humidification filter 533a. Accordingly, the air becomes humidified air.

The humidified air, having passed through the humidification element 533 and flowing obliquely upward to the left, is deflected to flow vertically upward by the right lateral surface 531b of the humidification tank 531 functioning as a guide surface of air. The humidified air is then blown out through the blow-out port 511b (see FIG 17).

It should be noted that the setting to turn on the humidification function has been explained above. On the other hand, when the humidification function is set to be turned off, the humidification element 533 is configured not to be rotated, and the air passing through the humidification element 533 is blown out through the blow-out port 511b while being hardly humidified.

### (4) Detachment of Humidification Unit and Air Purification Filter

Next, detachment of the humidification unit 530 and the air purification filter 520 will be explained mainly with reference to FIGS. 19 and 20. FIG 19 depicts a detachment work for detaching the humidification unit 530 and the air purification filter 520 from the casing 511. FIG 19 depicts the detachment work for the humidification unit 530 and the air purification filter 520 using a cross-sectional view of the interior of the casing 511 seen from the front side. FIG. 20 depicts a detachment work for detaching the humidification unit 530 from the casing 511. FIG. 20 depicts the detachment work for the humidification unit 530 using a perspective view of the casing 511 seen from the left front side.

FIGS. 19(a) and 20(a) show a condition of the air purifier 510 before starting the detachment work for the humidification unit 530 and the air purification filter 520.

In detaching the humidification unit 530 and the air purification filter 520, firstly, a user holds the pull 511ca of the first side plate 511c and, as shown in FIGS. 19(b) and 20(b), opens the first side plate 511c such that the upper part of the first side plate 511c is tilted to the left. Then, the user pulls out the humidification tank 531 integrated with the first side plate 511c obliquely upward to the left. Accordingly, the humidification tank 531 is detached from the casing 511 (see FIGS. 19(b) and 20(c)).

Next, as shown in FIGS. 19(c) and 20(d), the humidification tray 532 is detached from the casing 511. Specifically, the user holds the pull 511da provided in the second side plate 511d composing part of the humidification tray 532, and pulls out the humidification tray 532 to the user's side (the left side). At this time, the coupling part 533d of the rotor rotating shaft 533c of the humidification element 533 is detached from the coupling receiver 534b provided on the drive shaft 534a coupled to the humidification motor 534. Here, the coupling part 533d is only inserted into the hole 534ba of the coupling receiver 534b, and is not particularly fixed thereto by fixation members (e.g., screws). Therefore, the coupling part 533d can be easily pulled out from the hole 534ba (see FIG 16). Additionally, the guide pins 532f provided on the lateral surfaces of the humidification tray 532 are herein moved along the guides 511i formed on the inner surfaces of the casing 511. Hence, the user can smoothly pull out the humidification tray 532 horizontally to the left (see FIG. 16).

By thus detaching the humidification tray 532 from the casing 511 in the condition that the humidification element 533 is mounted to the shaft receiver 532e, the opening 515 is largely opened in the left surface of the casing 511 (see FIGS. 19(d) and 20(d)). Additionally, a large space is produced in the upper region of the interior of the casing 511 after detachment of the humidification tray 532 from the upper region. In other words, as a result of detachment of the humidification tray 532 and the humidification element 533 from the casing 511, a space in which the air purification filter 520 is movable is reliably produced in the humidification compartment S3.

Afterwards, as shown in FIG. 19(d), the deodorization filter 522 of the air purification filter 520 is firstly detached from the casing 511. Specifically, while holding the recess 524d on the top surface of the airflow guide 524 integrated with the filter case 523, the user upwardly lifts the filter case 523 from the support part 511f of the casing 511, and simultaneously, pulls out the protrusions 523a provided on the right surface of the filter case 523 from the recesses 511g of the casing 511. Next, while holding the recess 524d, the user moves the filter case 523 in the thickness direction (upward direction) of the deodorization filter 522, and then takes it out from the interior of the casing 511 by moving it through the humidification compartment S3. In other words, the deodorization filter 522 is lifted up to the downstream side in the flow direction of air, and is then taken out from the casing 511 through the opening 515.

Finally, as shown in FIG. 19(e), the dust collecting filter 521 of the air purification filter 520 is taken out from the casing 511. Specifically, the user lifts up the dust collecting filter 521 from the flanged part 511e of the casing 511, moves it in the thickness direction (upward direction) thereof, and then takes it out from the casing 511 by moving it through the humidification compartment S3. In other words, the dust collecting filter 521 is lifted up to the downstream side in the flow direction of air, and is then taken out from the casing 511 through the opening 515.

### (5) Attachment of Humidification Unit and Air Purification Filter

Next, attachment of the humidification unit 530 and the air purification filter 520 will be explained. It should be noted that the diagrams in FIGS. 19 and 20 depict an attachment work of the humidification unit 530 and the air purification filter 520 when seen in the reverse order. Therefore, diagrams will not be herein provided for depicting conditions of the attachment work of the humidification unit 530 and the air purification filter 520.

Attachment of the humidification unit 530 and the air purification filter 520 will be explained based on a condition that the air purification filter 520 and the humidification unit 530 have been all detached.

Firstly, as the first step, the dust collecting filter 521 of the air purification filter 520 is attached to the interior of the casing 511. Specifically, a user inserts the dust collecting filter 521 into the humidification compartment S3 through the opening 515, and then downwardly moves it to put it on the flanged part 511e of the casing 511 (see FIGS. 19(d) and 19(e)).

Next, the user attaches the deodorization filter 522 accommodated in the filter case 523 to a position above the dust collecting filter 521 in the casing 511.

Specifically, while holding the recess 524d of the airflow guide 524 integrated with the filter case 523, the user inserts the filter case 523 into the humidification compartment S3 through the opening 515, with the right part of the filter case 523 being tilted down. Afterwards, the user moves the filter case 523 from the left side to the right side, while causing the right part of the bottom surface of the filter case 523 to slide on the upper surface 521bb (see FIG 19(d)) of the circumferential edge 521b (see FIG 11) of the dust collecting filter 521. At this time, the user moves the filter case 523 from the left side to the right side, while gradually tilting down the left part of the filter case 523. The filter case 523 is moved rightward on the dust collecting filter 521 until the protrusions 523a provided on the filter case 523 are fitted to the recesses 511g provided on the casing 511. When the protrusions 523a are fitted to the recesses 511g, the left part of the filter case 523 is tilted down and can be put on the support part 511f. By contrast, when the protrusions 523a are not fitted to the recesses 511g, the left part of the filter case 523 interferes with the inner surface of the casing 511, and thus, the filter case 523 cannot be accommodated in the casing 511.

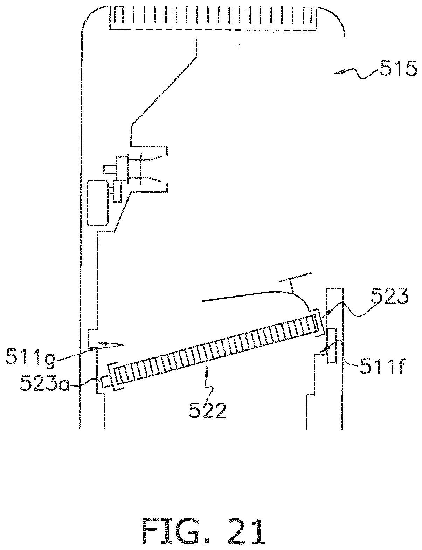

It should be noted that when it is assumed to attach the filter case 523 without attachment of the dust collecting filter 521, a structure for supporting the filter case 523 does not exist below the filter case 523, and a structure for guiding the protrusions 523a of the filter case 523 to the recesses 511g does not exist, too. Therefore, in trying to attach the filter case 523 without attachment of the dust collecting filter 521, it is inevitable for the right part of the filter case 523 to be easily displaced downward as shown in FIG 21, and it is difficult to attach the filter case 523 in an appropriate position.

In a condition that the deodorization filter 522 accommodated in the filter case 523 has been attached to the casing 511, in other words, in a condition that the protrusions 523a of the filter case 523 have been fitted to the recesses 511g of the casing 511 while the left part of the filter case 523 has been supported by the support part 511f, the filter case 523 is in contact with the upper surface 521bb (see FIG 7) of the circumferential edge 521b (see FIG. 11) of the dust collecting filter 521. As further described below, in a condition that the humidification tray 532 and the humidification tank 531 have been attached to the casing 511, the bottom surface 532b of the humidification tray 532 is in contact with the top surface 524c of the airflow guide 524 integrated with the filter case 523. The filter case 523 is restricted from moving upward by the humidification tray 532 and the humidification tank 531, and furthermore, the filter case 523 and the upper surface 521bb of the circumferential edge 521b of the dust collecting filter 521 are in contact with each other. Therefore, the dust collecting filter 521 is prevented from moving upward even when a force to float the dust collecting filter 521 acts thereon due to the flow of air.

When attachment of the air purification filter 520 has been finished, the humidification tray 532 is attached to the casing 511 (see FIGS. 19(c) and 20(d)). Specifically, while holding the pull 511 da provided on the second side plate 511 d composing part of the humidification tray 532, the user moves the humidification tray 532 horizontally to the right such that the guide pins 532f provided on the humidification tray 532 are fitted to the guides 511i provided on the front and rear inner walls of the casing 511 and the tip part 533e of the rotor rotating shaft 533c of the humidification element 533 is inserted into the hole 534ba of the coupling receiver 534b of the drive shaft 534a coupled to the humidification motor 534 (see FIG. 16). The guides 511i and the coupling receiver 534b herein function as guides, and accordingly, the humidification tray 532 can be easily moved in the horizontal direction. It should be noted that when the phase of the hexagonal coupling part 533d and that of the hexagonal hole 534ba are misaligned in inserting the coupling part 533d into the hole 534ba, the humidification element 533 is rotated such that the phase of the hexagonal coupling part 533d and that of the hexagonal hole 534ba are aligned. Accordingly, the coupling part 533d and the coupling receiver 534b are coupled to each other.

When the humidification tray 532 has been attached to the casing 511, the bottom surface 532b of the humidification tray 532 is in contact with the top surface 524c of the airflow guide 524 integrated with the filter case 523. With the condition that the humidification tray 532 and the top surface 524c of the airflow guide 524 are in contact with each other, the air purification filter 520 is prevented from moving upward even when a force to float the air purification filter 520 acts thereon due to the flow of air. In other words, the bottom surface 532b of the humidification tray 532 herein functions as the movement restricting part for restricting upward movement of the air purification filter 520.

Finally, while holding the pull 511ca of the first side plate 511c, the user causes the lower part of the first side plate 511c integrated with the humidification tank 531 to be engaged with the upper part of the second side plate 511d, with the upper part of the first side plate 511c being tilted to the left (see FIGS. 19(b) and 20(b)). Next, by making the first side plate 511c stand upright, the opening 515 of the casing 511 is closed (see FIGS. 19(a) and 20(a)).

### (6) Features

### (6-1)

The air purifier 510 according to the aforementioned embodiment is an air purifier having a humidity regulation function, and includes the air purification filter 520, the humidification element 533 and the fan 512a. The air purification filter 520 removes dust and dirt contained in air. The humidification element 533 is configured to humidify air by vaporizing water to be supplied thereto. The fan 512a is configured to blow air to the air purification filter 520 and the humidification element 533. In the air purifier 510, the fan 512a, the air purification filter 520 and the humidification element 533 are sequentially disposed from below in the order of the fan 512a, the air purification filter 520 and the humidification element 533.

In the air purifier 510, the air sucked by the fan 512a from the blowing compartment S1 located in a low position passes through the air purification filter 520 disposed in the air purification compartment S2 located in a high position, then passes through the humidification element 533 disposed in the humidification compartment S3 located in a higher position, and is blown out from the air purifier 510. The blower device 512 as a sound source is located in the lowermost position. Hence, sounds to be produced from the fan motor 512b and the fan 512a are attenuated by the air purification filter 520 and the humidification element 533. In other words, noises are inhibited.

### (6-2)

The air purifier 510 according to the aforementioned embodiment includes the casing 511 as an exemplary body casing. The casing 511 accommodates the air purification filter 520, the humidification element 533 and the fan 512a. Additionally, the casing 511 includes the blowing compartment S1, the air purification compartment S2, the humidification compartment S3, the first opening K1 and the second opening K2. The fan 512a is disposed in the blowing compartment S1. The air purification filter 520 is disposed in the air purification compartment S2. The humidification element 533 is disposed in the humidification compartment S3. The first opening K1 is an opening through which air flows from the blowing compartment S1 to the air purification compartment S2. The second opening K2 is an opening through which air flows from the air purification compartment S2 to the humidification compartment S3. The first opening K1 and the second opening K2 are displaced from each other in a top view.

In the air purifier 510 according to the aforementioned embodiment, static pressure acts on the entire surface of the air purification filter 520 by the air that has entered the air purification compartment S2, and the air passes through the air purification filter 520. The first opening K1, which is provided between the blowing compartment S1 and the air purification compartment S2, and the second opening K2, which is provided between the air purification compartment S2 and the humidification compartment S3, are displaced from each other in the top view. Hence, in comparison with a configuration that these openings are not displaced from each other, the speed of air exhibits a widely ranged speed distribution when air passes through the air purification filter 520. In other words, a part, through which air does not pass or in which the speed of air is extremely low, is reduced in the air purification filter 520.

### (6-3)

In the air purifier 510 according to the aforementioned embodiment, the first opening K1 is displaced to one side (left side) with respect to the center of the air purification filter 520, whereas the second opening K2 is displaced to the opposite side (right side) of the one side with respect to the center of the air purification filter 520.

The first opening K1 and the second opening K2 are herein displaced oppositely to each other with respect to the center of the air purification filter 520. Hence, in comparison with a configuration that these openings are not displaced from each other, the speed of air exhibits a widely ranged speed distribution when air passes through the air purification filter 520. In other words, a part, through which air does not pass or in which the speed of air is extremely low, is reduced in the air purification filter 520.

### (6-4)

The air purifier 510 according to the aforementioned embodiment further includes the fan motor 512b, which is disposed in the blowing compartment S1 and is configured to drive the fan 512a, and the humidification tank 531, which is disposed as an exemplary water storage tank in the humidification compartment S3 and is configured to supply water to the humidification element 533. The fan motor 512b is displaced to one side with respect to the center of the air purification filter 520, whereas the humidification tank 531 is displaced oppositely to the one side with respect to the center of the air purification filter 520.

In the present air purifier 510, the heavy fan motor 512b and the heavy humidification tank 531 are separated away from each other in opposite directions without being disposed only on the same side in a top view. Thus, the casing 511 has good upright stability.

### (6-5)

In the air purifier 510 according to the aforementioned embodiment, the air purification filter 520 has a horizontal projected area of greater than or equal to 80% of that of the air purification compartment S2. The first opening K1 has an area set to fall in a range of 20% to 30% of the horizontal projected area of the air purification compartment S2. The second opening K2 has an area set to fall in a range of 40% to 50% of the horizontal projected area of the air purification compartment S2.

Here, the overlapped region between the first opening K1 and the second opening K2 is eliminated or reduced by setting the second opening K2 to be larger than the first opening K1 and simultaneously by displacing the first opening K1 and the second opening K2 in a top view. Hence, the speed of air exhibits a widely ranged speed distribution when air passes through the air purification filter 520, and a part, through which air does not pass or in which the speed of air is extremely low, is reduced in the air purification filter 520. Accordingly, it is possible to make air evenly flow through the entire surface of the air purification filter 520.

### (6-6)

In the air purifier 510 according to the aforementioned embodiment, the fan 512a is a sirocco fan. Therefore, a stable airflow rate is achieved.

### (6-7)

The air purifier 510 according to the aforementioned embodiment further includes the airflow guide 524 disposed above the air purification filter 520. The airflow guide 524 includes the first guide surface 524b with a curved shape configured to direct the air having passed through the air purification filter 520 to the humidification element 533.

With the airflow guide 524 herein provided, the air having passed through the air purification filter 520 can be easily and smoothly directed to the humidification element 533, and also, the air can be directed to the humidification element 533 while inhibiting pressure loss.

### (6-8)

In the air purifier 510 according to the aforementioned embodiment, the airflow guide 524 is integrated with the filter case 523 that accommodates a part of the air purification filter 520, herein specifically, the deodorization filter 522.

Here, the filter case 523 accommodating the air purification filter 520 and the airflow guide 524 are integrated. Hence, the air having passed through the air purification filter 520 can be easily directed to the humidification element 533 without being allowed to flow through another path.

### (6-9)

The air purifier 510 according to the aforementioned embodiment further includes, as an exemplary water retention tray, the humidification tray 532 which is disposed below the humidification element 533 and is configured to retain water to be supplied to the humidification element 533. The humidification tray 532 includes the second guide surface 532a with a curved shape configured to direct the air having passed through the air purification filter 520 to the humidification element 533, on the lower part thereof.

With the second guide surface 532a herein provided on the lower part of the humidification tray 532, the air having passed through the air purification filter 520 can be easily and smoothly directed to the humidification element 533, and also, the air can be directed to the humidification element 533 while inhibiting pressure loss.

Especially, in the air purifier 510 according to the aforementioned embodiment, the first guide surface 524b of the airflow guide 524 and the second guide surface 532a of the humidification tray 532 form a continuous curve through the gap G (the gap between the airflow guide 524 and the humidification tray 532).

In the present air purifier 510, with a continuous curve formed by the first guide surface 524b and the second guide surface 532a, the air having passed through the air purification filter 520 can be easily and smoothly directed to the humidification element 533, and also, the air can be directed to the humidification element 533 while inhibiting pressure loss.

### (6-10)

In the air purifier 510 according to the aforementioned embodiment, the casing 511 includes the bypass openings 511ha bored above the second opening K2 so as to direct air to the outside of the casing 511 from the humidification compartment S3 without passing the air through the humidification element 533.

Here, as the bypass openings 511ha are bored above the second opening K2, part of air can be directed outside the casing 511 through the bypass openings 511ha, while pressure loss is hardly caused. Therefore, it is easy to make air flow through the air purification filter 520 at a large airflow rate.

Moreover, the first opening K1 and the second opening K2 are herein displaced from each other in a top view. Hence, it is possible to make air evenly flow through the entire surface of the air purification filter 520 at a large airflow rate, and thus, it is possible to efficiently perform a processing of removing dust and dirt from air.

### (7) Modifications

Modifications of the air purifier 510 according to the second embodiment will be hereinafter described. It should be noted that these modifications may be applied to the air purifier 10 according to the first embodiment as long as contradictions are not caused in applying the modifications to the air purifier 10.

### (7-1) Modification A

In the aforementioned embodiment, the airflow guide 524 is integrated with the filter case 523. However, the configuration of the airflow guide 524 is not limited to this. For example, the airflow guide 524 may be provided on the bottom surface 532b of the humidification tray 532. Alternatively, for instance, the airflow guide 524 may be provided separately from the filter case 523 and the humidification tray 532.

It should be noted that when the airflow guide 524 is provided on the bottom surface of the humidification tray 532, the opening 515 of the casing 511 is required to be produced with a large size. Hence, the airflow guide 524 is preferably integrated with the filter case 523.

Additionally, from the perspectives of reduction in number of components and maintenance performance, the airflow guide 524 is preferably integrated with the filter case 523 and/or so forth without being provided as a separate member. Moreover, when the airflow guide 524 is provided as a separate member, gaps causing air to pass therethrough are likely to be produced, and finally, the air is likely to flow out to the outside through gaps in the casing and so forth. Hence, the airflow guide 524 is more preferably integrated with the filter case 523 and/or so forth without being provided as a separate member.

### (7-2) Modification B

In the aforementioned embodiment, the air purifier 510 includes the first guide surface 524b and the second guide surface 532a. However, the configuration of the air purifier 510 is not limited to this. The air purifier 510 may include only either of the first guide surface 524b and the second guide surface 532a. It should be noted that both of the first guide surface 524b and the second guide surface 532a are preferably provided for directing the air, having passed through the air purification filter 520, to the humidification element 533 and simultaneously for inhibiting pressure loss.

### (7-3) Modification C

In the aforementioned embodiment, the bypass openings 511ha are provided in the baffle plate 511h of the casing 511. However, the configuration of the bypass openings 511ha is not limited to this, and alternatively, the bypass openings 511ha may not be provided. It should be noted that the bypass openings 511ha are preferably provided for increasing the amount of air passing through the air purification filter 520 and by efficiently purifying the air.

### (7-4) Modification D

In the aforementioned embodiment, the air purification filter 520 includes the deodorization filter 522 as well. However, the configuration of the air purification filter 520 is not limited to this. The air purification filter 520 may include only the dust collecting filter 521. In this case, the dust collecting filter 521 may be designed to be accommodated in the filter case 523.

### (7-5) Modification E

In the aforementioned embodiment, the filter case 523 is provided with the protrusions 523a whereas the casing 511 is provided with the recesses 511g, and the protrusions 523a are fitted to the recesses 511g. However, the configuration of the protrusions 523a and the recesses 511g is not limited to this. For example, the filter case 523 may be provided with recesses whereas the casing 511 may be provided with protrusions, and the protrusions of the casing 511 may be designed to be fitted to the recesses of the filter case 523.

### (7-6) Modification F

In the aforementioned embodiment, the configuration employed for the humidification unit 530 is of a type that the humidification filter 533a of the humidification element 533 is immersed into the water in the water supply part 532d of the humidification tray 532. However, the configuration to be employed for the humidification unit 530 is not limited to this.

For example, a configuration of a ladle equipped humidification rotor type may be employed for the humidification unit 530.

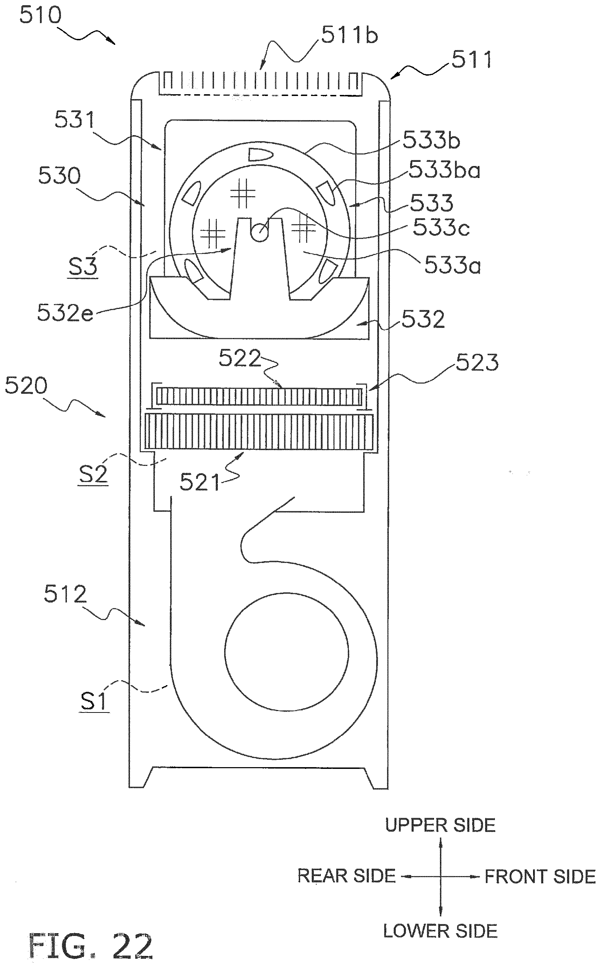

Specifically, in the humidification unit 530 with a configuration of a ladle equipped humidification rotor type, the humidification rotor 533b of the humidification element 533 is provided with a plurality of ladles 533ba (see FIG 22). In the configuration of a ladle equipped humidification rotor type, the humidification filter 533a is not configured to be directly immersed into the water, but is alternatively configured to scoop water with the ladles 533ba in the water supply part 532d of the humidification tray 532 when the humidification rotor 533b is rotated. When the humidification rotor 533b is then further rotated and a given one of the ladles 533ba containing the scooped water is moved nearby the highest position, the given one of the ladles 533ba is configured to pour the scooped water to the humidification filter 533a. Thus, water is supplied to the humidification filter 533a.

### (7-7) Modification G

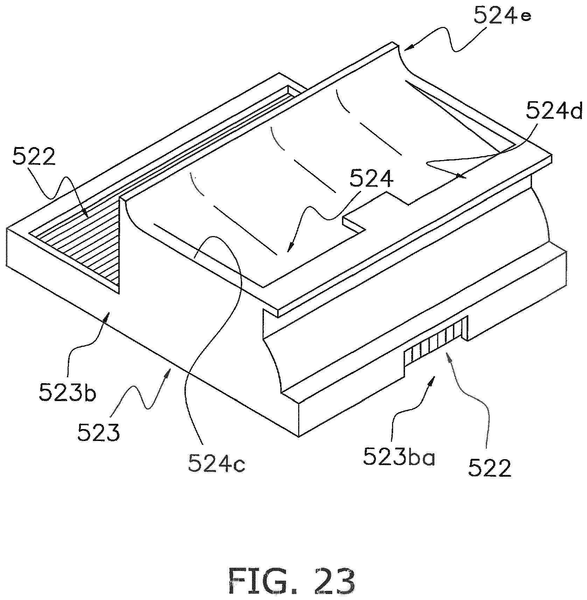

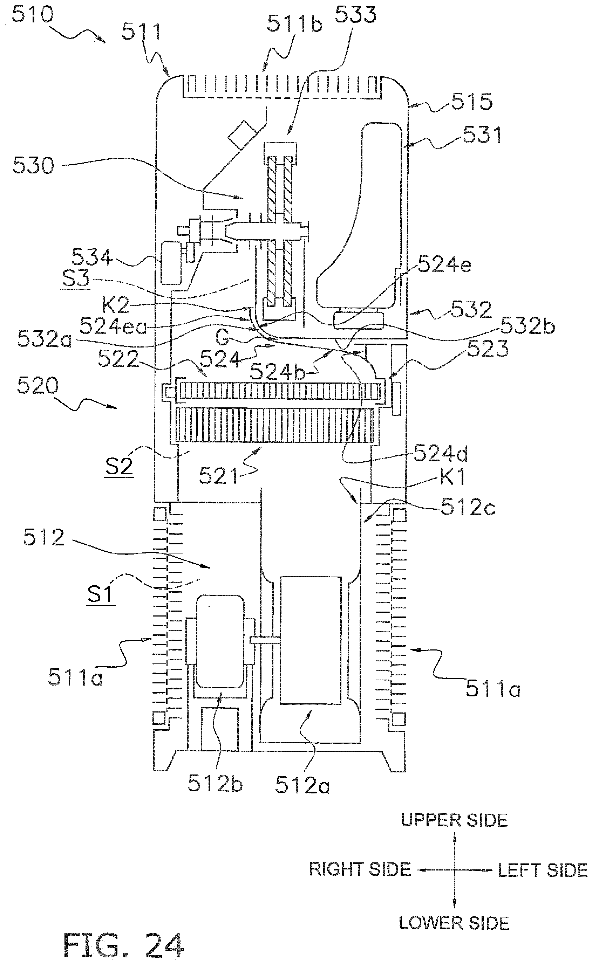

The shape of the airflow guide 524 in the aforementioned embodiment is exemplary only, and is not limited to this. For example, the airflow guide 524 may include an extended part 524e shaped for covering a second opening K2-side lower part of the humidification tray 532, more specifically, a lower edge of the humidification tray 532 that is located adjacently to the second opening K2 (see FIGS. 23 and 24). The left lateral surface of the extended part 524e is opposed to the lower edge of the humidification tray 532, which is located adjacently to the second opening K2, and the lower part of the right lateral surface of the humidification tray 532. The right lateral surface of the extended part 524e functions as an extended guide surface 524ea with a curved shape configured to direct the air, having passed through the air purification filter 520, to the humidification element 533 (see FIG. 24). The extended guide surface 524ea forms a continuous curve together with the first guide surface 524b (see FIG 24).

With this configuration, it is easy to prevent the air, having passed through the air purification filter 520, from flowing into the gap G produced between the airflow guide 524 and the humidification tray 532. Additionally, while pressure loss is inhibited, air can be directed to the humidification element 533 by the first guide surface 524b and the extended guide surface 524ea, which is provided downstream of the first guide surface 524b in the flow direction of air and forms a continuous curve together with the first guide surface 524b.

### INDUSTRIAL APPLICABILITY

As described above, according to the present invention, sounds produced from the fan motor 26 and the fan rotor 25 are attenuated by the air purification filter 22 and the humidification element 24. Additionally, according to the present invention, sounds produced from the fan motor 512b and the fan 512a are attenuated by the air purification filter 520 and the humidification element 533. This is also applicable to dehumidifiers using a hygroscopic element

### REFERENCE SIGNS LIST

- 10, 510: Air purifier
- 22, 520: Air purification filter
- 24, 533: Humidification element
- 25: Fan rotor (fan)
- 26, 512b: Fan motor
- 31: Body casing
- 31a, S1: Blowing compartment
- 31b, S2: Air purification compartment
- 31c, S3: Humidification compartment
- 40: Tank (water storage tank)
- 311, K1: First opening
- 312, K2: Second opening
- 511: Casing (body casing)
- 511ha: Bypass opening
- 512a: Fan
- 523: Filter case
- 524: Airflow guide
- 524b: First guide surface
- 531: Humidification tank (water storage tank)
- 532: Humidification tray (water retention tray)
- 532a: Second guide surface
- G: Gap

### CITATION LIST

### PATENT LITERATURE

PTL 1: Japan Laid-open Patent Application Publication No. 2010-17685

## Claims

1. An air purifier (10, 510) having a humidity regulation function, comprising:
an air purification filter (22, 520) configured to remove dust and dirt contained in air;
a humidification element (24, 533) configured to humidify the air by vaporizing water to be supplied thereto; and
a fan (25, 512a) configured to blow the air to the air purification filter (22, 520) and the humidification element (24, 533),
wherein the air purifier (10, 510) further comprises a body casing (31, 511) accommodating the air purification filter (22, 520), the humidification element (24, 533) and the fan (25, 512a), **characterized in that**
the fan (25, 512a), the air purification filter (22, 520) and the humidification element (24, 533) are sequentially disposed from below in an order of the fan (25, 512a), the air purification filter (22, 520) and the humidification element (24, 533), and **in that**
the body casing (31, 511) includes
a blowing compartment (31a, Si) in which the fan (25, 512a) is disposed,
an air purification compartment (31b, S2) in which the air purification filter (22, 520) is disposed,
a humidification compartment (31c, S3) in which the humidification element (24,533) is disposed,
a first opening (311, K1) through which the air flows from the blowing compartment (31a, S1) to the air purification compartment (31b, S2), and
a second opening (312, K2) through which the air flows from the air purification compartment (31b, S2) to the humidification compartment (31c, S3), wherein
the first opening (311, K1) and the second opening (312, K2) are displaced from each other in a top view.

2. The air purifier (10, 510) according to claim 1, wherein
the first opening (311, K1) is displaced to one side with respect to a center of the air purification filter (22, 520), and
the second opening (312, K2) is displaced oppositely to the one side with respect to the center of the air purification filter (22, 520).

3. The air purifier (10, 510) according to claim 1, further comprising:
a fan motor (26, 512b) disposed in the blowing compartment (31a, S1), the fan motor (26, 512b) being configured to drive the fan (25, 512a), and
a water storage tank (40, 531) disposed in the humidification compartment (31c, S3), the water storage tank (40, 531) being configured to supply the water to the humidification element (24, 533), wherein the fan motor (26, 512b) is displaced to one side with respect to a center of the air purification filter (22, 520), and
the water storage tank (40, 531) is displaced oppositely to the one side with respect to the center of the air purification filter (22, 520).

4. The air purifier (10, 510) according to claim 1, wherein
the air purification filter (22, 520) has a horizontal projected area of greater than or equal to 80% of a horizontal projected area of the air purification compartment (31b, S2),
the first opening (311, K1) has an area set to fall in a range of 20% to 30% of the horizontal projected area of the air purification compartment (31b, S2), and
the second opening (312, K2) has an area set to fall in a range of 40% to 50% of the horizontal projected area of the air purification compartment (31b, S2).

5. The air purifier (510) according to claim1 , wherein the body casing (511) includes a bypass opening (511ha) bored above the second opening (K2) so as to direct the air to outside of the body casing (511) from the humidification compartment (S3) without passing the air through the humidification element (533).

6. The air purifier (10,510) according to claim 1, wherein the fan (25, 512a) is a sirocco fan.

7. The air purifier (510) according to any one of claims 1 to 6, further comprising:
an airflow guide (524) disposed above the air purification filter (520), wherein the airflow guide (524) includes a first guide surface (524b) with a curved shape configured to direct the air having passed through the air purification filter (520) to the humidification element (533).

8. The air purifier (510) according to claim 7, further comprising a filter case (523) accommodating at least part of the air purification filter (520), wherein the airflow guide (524) is integrated in the filter case (523).

9. The air purifier (510) according to claim 7 or 8, further comprising:
a water retention tray (532) configured to retain the water to be supplied to the humidification element (533), the water retention tray (532) being disposed below the humidification element (533), wherein the water retention tray (532) includes a second guide surface (532a) with a curved shape on a lower part thereof, the second guide surface (532a) being configured to direct the air having passed through the air purification filter (520) to the humidification element (533).

10. The air purifier (510) according to claim 9, wherein
the first guide surface (524b) and the second guide surface (532a) form a continuous curve through a gap (G) produced therebetween.

11. The air purifier (510) according to any one of claims 1 to 6, further comprising:
a water retention tray (532) configured to retain the water to be supplied to the humidification element (533), the water retention tray (532) being disposed below the humidification element (533), wherein
the water retention tray (532) includes a second guide surface (532a) with a curved shape on a lower part thereof, the second guide surface ((532a) being configured to direct the air having passed through the air purification filter (520) to the humidification element (533).

12. The air purifier (510) according to claim 7 or 8, further comprising:
a water retention tray (532) configured to retain the water to be supplied to the humidification element (533), the water retention tray (532) being disposed below the humidification element (533), wherein the airflow guide (524) includes an extended part (524e) shaped for covering a second opening (K2)-side lower part of the water retention tray (532),
the extended part (524e) includes an extended guide surface (524ea) with a curved shape configured to direct the air having passed through the air purification filter (520) to the humidification element (533), and
the extended guide surface (524ea) forms a continuous curve together with the first guide surface (524b).

## Patentansprüche

1. Luftreiniger (10, 510) mit einer Feuchtigkeitsregulierungsfunktion, umfassend:
einen Luftreinigungsfilter (22, 520), der konfiguriert ist, um Staub und Schmutz, die in der Luft enthalten sind, zu entfernen;
ein Befeuchtungselement (24, 533), das konfiguriert ist, um die Luft durch Verdampfen von Wasser, das ihr zugeführt wird, zu befeuchten; und
ein Gebläse (25, 512a), das konfiguriert ist, um die Luft zu dem Luftreinigungsfilter (22, 520) und dem Befeuchtungselement (24, 533) zu blasen,
wobei der Luftreiniger (10, 510) ferner ein Körpergehäuse (31, 511) umfasst, das den Luftreinigungsfilter (22, 520), das Befeuchtungselement (24, 533) und das Gebläse (25, 512a) aufnimmt,
**dadurch gekennzeichnet, dass** das Gebläse (25, 512a), der Luftreinigungsfilter (22, 520) und das Befeuchtungselement (24, 533) nacheinander von unten in einer Reihenfolge des Gebläses (25, 512a), des Luftreinigungsfilters (22, 520) und des Befeuchtungselements (24, 533) angeordnet sind,
und dass
das Körpergehäuse (31, 511) eine Gebläsekammer (31a, S1), in dem das Gebläse (25, 512a) angeordnet ist,
eine Luftreinigungskammer (31b, S2), in der der Luftreinigungsfilter (22, 520) angeordnet ist,
eine Befeuchtungskammer (31c, S3), in der das Befeuchtungselement (24, 533) angeordnet ist,
eine erste Öffnung (311, K1), durch die die Luft von der Gebläsekammer (31a, S1) zu der Luftreinigungskammer (31b, S2) strömt, und
eine zweite Öffnung (312, K2) beinhaltet, durch die die Luft von der Luftreinigungskammer (31b, S2) zu der Befeuchtungskammer (31c, S3) strömt, wobei die erste Öffnung (311, K1) und die zweite Öffnung (312, K2) in Draufsicht gegeneinander verschoben sind.

2. Luftreiniger (10, 510) nach Anspruch 1, wobei
die erste Öffnung (311, K1) zu einer Seite in Bezug auf eine Mitte des Luftreinigungsfilters (22, 520) versetzt ist, und
die zweite Öffnung (312, K2) in entgegengesetzter Richtung zu der einen Seite in Bezug auf die Mitte des Luftreinigungsfilters (22, 520) versetzt ist.

3. Luftreiniger (10, 510) nach Anspruch 1, ferner umfassend:
einen Gebläsemotor (26, 512b), der in der Gebläsekammer (31a, S1) angeordnet ist,
wobei der Gebläsemotor (26, 512b) zum Antreiben des Gebläses (25, 512a) konfiguriert ist, und
einen Wasserspeichertank (40, 531), der in der Befeuchtungskammer (31c, S3) angeordnet ist, wobei der Wasserspeichertank (40, 531) konfiguriert ist, um das Wasser dem Befeuchtungselement (24, 533) zuzuführen, wobei der Gebläsemotor (26, 512b) in Bezug auf eine Mitte des Luftreinigungsfilters (22, 520) zu einer Seite versetzt ist, und
der Wasserspeichertank (40, 531) in entgegengesetzter Richtung zu der einen Seite in Bezug auf die Mitte des Luftreinigungsfilters (22, 520) versetzt ist.

4. Luftreiniger (10, 510) nach Anspruch 1, wobei
der Luftreinigungsfilter (22, 520) eine horizontal projizierte Fläche von 80% oder mehr einer horizontal projizierten Fläche der Luftreinigungskammer (31b, S2) aufweist, wobei die erste Öffnung (311, K1) eine Fläche aufweist, die in einen Bereich von 20% bis 30% der horizontal projizierten Fläche der Luftreinigungskammer (31b, S2) fällt, und wobei die zweite Öffnung (312, K2) eine Fläche aufweist, die in einen Bereich von 40% bis 50% der horizontal projizierten Fläche der Luftreinigungskammer (31b, S2) fällt.

5. Luftreiniger (510) nach Anspruch 1, wobei das Körpergehäuse (511) eine Bypassöffnung (511ha) aufweist, die oberhalb der zweiten Öffnung (K2) gebohrt ist, um die Luft von der Befeuchtungskammer (S3) aus dem Körpergehäuse (511) heraus zu leiten, ohne die Luft durch das Befeuchtungselement (533) zu leiten.

6. Luftreiniger (10,510) nach Anspruch 1, wobei das Gebläse (25,512a) ein Sirocco-Ventilator ist.

7. Luftreiniger (510) nach einem der Ansprüche 1 bis 6, ferner umfassend:
eine Luftstromführung (524), die oberhalb des Luftreinigungsfilters (520) angeordnet ist, wobei die Luftstromführung (524) eine erste Führungsfläche (524b) mit einer gekrümmten Form aufweist, die konfiguriert ist, um die durch den Luftreinigungsfilter (520) geströmte Luft zu dem Befeuchtungselement (533) zu leiten.

8. Luftreiniger (510) nach Anspruch 7, ferner umfassend ein Filtergehäuse (523), das mindestens einen Teil des Luftreinigungsfilters (520) aufnimmt, wobei die Luftstromführung (524) in das Filtergehäuse (523) integriert ist.

9. Luftreiniger (510) nach Anspruch 7 oder 8, ferner umfassend:
eine Wasserrückhaltewanne (532), die konfiguriert ist, um das dem Befeuchtungselement (533) zuzuführende Wasser zurückzuhalten, wobei die Wasserrückhaltewanne (532) unterhalb des Befeuchtungselements (533) angeordnet ist, wobei die Wasserrückhaltewanne (532) eine zweite Führungsfläche (532a) mit einer gekrümmten Form an einem unteren Teil davon aufweist, wobei die zweite Führungsfläche (532a) konfiguriert ist, um die durch den Luftreinigungsfilter (520) geströmte Luft zum Befeuchtungselement (533) zu leiten.

10. Luftreiniger (510) nach Anspruch 9, wobei
die erste Führungsfläche (524b) und die zweite Führungsfläche (532a) eine kontinuierliche Kurve durch einen zwischen ihnen erzeugten Spalt (G) bilden.

11. Luftreiniger (510) nach einem der Ansprüche 1 bis 6, ferner umfassend:
eine Wasserrückhaltewanne (532), die konfiguriert ist, um das dem Befeuchtungselement (533) zuzuführende Wasser zurückzuhalten, wobei die Wasserrückhaltewanne (532) unterhalb des Befeuchtungselements (533) angeordnet ist, wobei
die Wasserrückhaltewanne (532) eine zweite Führungsfläche (532a) mit einer gekrümmten Form an einem unteren Teil davon beinhaltet, wobei die zweite Führungsfläche (532a) konfiguriert ist, um die Luft, die durch den Luftreinigungsfilter (520) geleitet wurde, zum Befeuchtungselement (533) zu leiten.

12. Luftreiniger (510) nach Anspruch 7 oder 8, ferner umfassend:
eine Wasserrückhaltewanne (532), die konfiguriert ist, um das dem Befeuchtungselement (533) zuzuführende Wasser zurückzuhalten, wobei die Wasserrückhaltewanne (532) unterhalb des Befeuchtungselements (533) angeordnet ist, wobei die Luftstromführung (524) einen verlängerten Teil (524e) beinhaltet, der zum Abdecken einer zweiten Öffnung an einem (K2) -seitigen unteren Teil der Wasserrückhaltewanne (532) geformt ist,
wobei der verlängerte Teil (524e) eine verlängerte Führungsfläche (524ea) mit einer gekrümmten Form aufweist, die konfiguriert ist, um die Luft, die durch den Luftreinigungsfilter (520) geleitet wurde, zum Befeuchtungselement (533) zu leiten, und
wobei die verlängerte Führungsfläche (524ea) zusammen mit der ersten Führungsfläche (524b) eine kontinuierliche Kurve bildet.

## Revendications

1. Épurateur d'air (10, 510) exerçant une fonction de régulation de l'humidité, comprenant :
un filtre d'épuration de l'air (22, 520) configuré pour extraire la poussière et les impuretés contenues dans l'air ;
un élément d'humidification (24, 533) configuré pour humidifier l'air par la vaporisation de l'eau à introduire dans celui-ci ; et
un ventilateur (25, 512a) configuré pour souffler l'air dans le filtre d'épuration de l'air (22, 520) et l'élément d'humidification (24, 533),
l'épurateur d'air (10, 510) comprenant en outre un boîtier de corps (31, 511) contenant le filtre d'épuration de l'air (22, 520), l'élément d'humidification (24, 533), et le ventilateur (25, 512a),
**caractérisé en ce que**
le ventilateur (25, 512a), le filtre d'épuration de l'air (22, 520) et l'élément d'humidification (24, 533) sont disposés en séquence, en partant du bas, dans l'ordre suivant : le ventilateur (25, 512a), le filtre d'épuration de l'air (22, 520) et l'élément d'humidification (24, 533),
et **en ce que**
le boîtier de corps (31, 511) comprend
un compartiment de soufflage (31a, S1) dans lequel est disposé le ventilateur (25, 512a),
un compartiment d'épuration de l'air (31b, S2) dans lequel est disposé le filtre d'épuration de l'air (22, 520),
un compartiment d'humidification (31c, S3) dans lequel est disposé l'élément d'humidification (24, 533),
une première ouverture (311, K1) à travers laquelle s'écoule l'air du compartiment de soufflage (31a, S1) au compartiment d'épuration de l'air (31b, S2), et
une deuxième ouverture (312, K2) à travers laquelle s'écoule l'air du compartiment d'épuration de l'air (31b, S2) au compartiment d'humidification (31c, S3),
la première ouverture (311, K1) et la deuxième ouverture (312, K2) étant déplacées l'une de l'autre dans une vue du dessus.

2. Épurateur d'air (10, 510) selon la revendication 1, dans lequel
la première ouverture (311, K1) est déplacée d'un côté relativement à un centre du filtre d'épuration de l'air (22, 520), et
la deuxième ouverture (312, K2) est déplacée de façon opposée à l'autre côté relativement au centre du filtre d'épuration de l'air (22, 520).

3. Épurateur d'air (10, 510) selon la revendication 1, comprenant en outre :
un moteur de ventilateur (26, 512b) disposé dans le compartiment de soufflage (31a, S1), le moteur de ventilateur (26, 512b) étant configuré pour entraîner le ventilateur (25, 512a), et
une cuve d'eau (40, 531) disposée dans le compartiment d'humidification (31c, S3), la cuve d'eau (40, 531) étant configurée pour alimenter en eau l'élément d'humidification (24, 533), le moteur de ventilateur (26, 512b) étant déplacé sur un côté relativement à un centre du filtre d'épuration de l'air (22, 520), et
la cuve d'eau (40, 531) étant déplacée de façon opposée au côté relativement au centre du filtre d'épuration de l'air (22, 520).

4. Épurateur d'air (10, 510) selon la revendication 1, dans lequel
le filtre d'épuration de l'air (22, 520) possède une surface saillante horizontale supérieure ou égale à 80% d'une surface saillante horizontale du compartiment d'épuration de l'air (31b, S2),
la première ouverture (311, K1) possède une surface réglée pour être comprise dans une plage allant de 20% à 30% de la surface saillante horizontale du compartiment d'épuration de l'air (31b, S2), et
la deuxième ouverture (312, K2) possède une surface réglée pour être comprise dans une plage allant de 40% à 50% de la surface saillante horizontale du compartiment d'épuration de l'air (31b, S2).

5. Épurateur d'air (510) selon la revendication 1, dans lequel le boîtier de corps (511) comprend une ouverture de dérivation (511ha) alésée au-dessus de la deuxième ouverture (K2) de façon à diriger l'air vers l'extérieur du boîtier de corps (511) depuis le compartiment d'humidification (S3) sans faire passer l'air à travers l'élément d'humidification (533).

6. Épurateur d'air (10, 510) selon la revendication 1, dans lequel le ventilateur (25, 512a) étant un ventilateur sirocco.

7. Épurateur d'air (510) selon une quelconque des revendications 1 à 6, comprenant en outre :
un guide de flux d'air (524) disposé au-dessus du filtre d'épuration de l'air (520), le guide de flux d'air (524) comprenant une première surface de guidage (524b) avec une forme courbe configurée pour diriger vers l'élément d'humidification (533) l'air qui est passé à travers le filtre d'épuration de l'air (520).

8. Épurateur d'air (510) selon la revendication 7, comprenant en outre un boîtier de filtre (523) contenant au moins une partie du filtre d'épuration de l'air (520), le guide de flux d'air (524) étant intégré dans le boîtier de filtre (523).

9. Épurateur d'air (510) selon la revendication 7 ou 8, comprenant en outre :
un plateau de retenue de l'eau (532) configuré pour retenir l'eau à fournir à l'élément d'humidification (533), le plateau de retenue de l'eau (532) étant disposé sous l'élément d'humidification (533), le plateau de retenue de l'eau (532) comprenant une deuxième surface de guidage (532a), de forme courbe, sur une partie inférieure de celle-ci, la deuxième surface de guidage (532a) étant configurée pour diriger vers l'élément d'humidification (533) l'air qui est passé à travers le filtre d'épuration de l'air (520).

10. Épurateur d'air (510) selon la revendication 9, dans lequel
la première surface de guidage (524b) et la deuxième surface de guidage (532a) forment une courbe continue à travers un écart (G) réalisé entre celles-ci.

11. Épurateur d'air (510) selon une quelconque des revendications 1 à 6, comprenant :
un plateau de retenue d'eau (532) configuré pour retenir l'eau devant être fournie à l'élément d'humidification (533), le plateau de retenue de l'eau (532) étant disposé sous l'élément d'humidification (533), dans lequel
le plateau de retenue d'eau (532) comprenant une deuxième surface de guidage (532a), de forme courbe, sur une partie inférieure de celui-ci, la deuxième surface de guidage (532a) étant configurée pour diriger vers l'élément d'humidification (533) l'air qui est passé à travers le filtre d'épuration de l'air (520).

12. Épurateur d'air (510) selon la revendication 7 ou 8, comprenant en outre :
un plateau de retenue de l'eau (532) configuré pour retenir l'eau à fournir à l'élément d'humidification (533), le plateau de retenue de l'eau (532) étant disposé sous l'élément d'humidification (533), le guide de flux d'air (524) comprenant une partie saillante (524e) façonnée de façon à couvrir une deuxième partie inférieure côté ouverture (K2) du plateau de retenue de l'eau (532),
la partie saillante (524e) comprenant une surface de guidage saillante (524ea), de forme courbe, configurée pour diriger vers l'élément d'humidification (533) l'air qui est passé à travers le filtre d'épuration de l'air (520), et
la surface de guidage saillante (524ea) formant une courbe continue conjointement avec la première surface de guidage (524b).
